# EUROPEAN PATENT SPECIFICATION

(11) **EP 3 387 444 B1**
(45) Date of publication and mention of the grant of the patent: **05.07.2023**
(21) Application number: 16809763.2
(22) Date of filing: 07.12.2016
(51) Int. Cl.: G01N 33/68, G01N 33/92

(54) **EXTRACELLULAR VESICLE MARKERS FOR STABLE ANGINA AND UNSTABLE ANGINA**
EXTRAZELLULÄRE VESIKELMARKER FÜR STABILE ANGINA UND INSTABILE ANGINA
MARQUEURS DE VÉSICULES EXTRACELLULAIRES POUR ANGINA STABLE ET INSTABLE

(30) Priority: 08.12.2015 NL 2015924
(43) Date of publication of application: 17.10.2018
(73) Proprietor: UMC Utrecht Holding B.V., 3584 CS Utrecht (NL)
(72) Inventor: DE KLEIJN, Dominicus Paschalis Victor, 3962 ET Wijk bij Duurstede (NL); TIMMERS, Leonardus, 3572 VA Utrecht (NL)
(74) Representative: De Vries & Metman
(86) International application number: PCT/EP2016/080131
(87) International publication number: WO 2017/097854

(56) References cited:
- EP-A1- 2 535 424
- WO-A1-2011/083145
- WO-A2-2012/110253
- TOUSOULIS ET AL: "Differences in inflammatory and thrombotic markers between unstable angina and acute myocardial infarction", INTERNATIONAL JOURNAL OF CARDIOLOGY, ELSEVIER, AMSTERDAM, NL, vol. 115, no. 2, 25 January 2007 (2007-01-25), pages 203-207, XP005858414, ISSN: 0167-5273, DOI: 10.1016/J.IJCARD.2006.03.011
- Vince C De Hoog: "Serum extracellular vesicle protein levels are associated with acute coronary syndrome", European Heart Journal Acute Cardiovascular Care, vol. 2 1 January 2012 (2012-01-01), 2012, pages 53-60, XP055337665, Retrieved from the Internet: URL:https://www.ncbi.nlm.nih.gov/pmc/artic les/PMC3760575/pdf/10.1177_204887261247121 2.pdf [retrieved on 2017-01-23]

## Description

### FIELD OF THE INVENTION

The invention relates to the field of medicine. More in particular it relates to a method for identifying a subject suffering from, or being at risk of suffering from, stable angina by measuring the concentration or a value related thereto of one or more protein markers in one or more blood plasma fractions. The protein markers are especially suitable for identifying a subject suffering from, or being at risk of suffering from, stable angina, and equivalents thereof.

### BACKGROUND ART

Ischemic heart disease is a major cause of morbidity and mortality worldwide. Although therapy for ischemic heart disease has greatly improved and mortality has gradually declined in industrialized countries during the last decades, mortality from ischemic heart disease is still rising in other parts of the world such as Africa and parts of Asia (Mathers and Loncar. Projections of global mortality and burden of disease from 2002 to 2030. PLoS Med. 2006;3:e442; WHO. World Health Statistics 2013. Geneva, Switserland; Alwan A. WHO. Global status report on noncommunicable diseases 2010. Geneva, Switserland). Clinical syndromes associated with ischemic heart disease are stable angina, unstable angina, and myocardial infarction.

### Stable angina

Stable coronary artery disease is the underlying cause of the clinical syndrome referred to as 'stable angina': episodes of stress-, exercise- or emotion-induced chest pain. Stable coronary artery disease can also cause atypical symptoms such as shortness of breath or reduced exercise tolerance. These symptoms are not generally referred to as stable angina, but can be referred to as 'stable angina equivalents' as they are caused by the same underlying disorder. Stable coronary artery disease is generally characterized by episodes of reversible myocardial demand/supply mismatch due to significant stenosis in the coronary arteries. Because the appearance of chest pain or equivalent symptoms are in a sense predictable, the disease is referred to as *stable* angina. The terms `stable angina' and 'stable coronary artery disease' are often mixed and often used in the art to mean the same thing. For clarity reasons, `stable angina' as used herein refers to all symptoms (predictable episodes of stress-, exercise- or emotion-induced chest pain, and atypical symptoms such as shortness of breath or reduced exercise tolerance) that are caused by stable coronary artery disease. The prevalence of stable angina varies from 5-14% depending on gender and age. Annual incidence of death is 1.2-2.4% (vs 0.6% in subjects without obstructive coronary artery disease), and the annual incidence of myocardial infarction is 2.7%. Because of the variety in clinical presentation and a broad differential diagnosis (such as musculoskeletal or psychological problems, pulmonary embolism, pneumonia, pneumothorax, pericarditis), the diagnosis of stable angina is notoriously challenging. Patients can be referred for exercise ECG or non-invasive imaging such as cardiac CT, MRI or nuclear scan. Such imaging tests have a sensitivity and specificity of around 85%, and are time consuming and expensive. In many cases, invasive coronary angiogram is needed to confirm the diagnosis and to determine the options for revascularisation by percutaneous coronary intervention or coronary artery bypass grafting. The diagnostic work-up is inefficient and expensive. A rapid straightforward test for diagnosing (or ruling out) stable angina is currently non-existent.

### Unstable angina

Annually, millions of patients enter the emergency rooms with chest pain or other symptoms that are suggestive of Acute Coronary Syndrome (ACS). ACS is a life threatening condition mostly caused by intracoronary thrombus formation leading to acute luminal narrowing or even occlusion. There are two types of ACS: unstable angina and myocardial infarction. Myocardial infarction can be quickly diagnosed on ECG and/or elevation of cardiac troponin in the blood. Unstable angina is an unstable coronary syndrome, without signs of myocardial injury (such as an elevated level of cardiac troponin). The term *unstable* is used because - in contrast to stable angina - the chest pain is not so much predictable and can also occur in rest and generally increases in severity over time. 5% of all people entering the emergency rooms with chest pain or other symptoms that suggest ACS, have unstable angina. Rapid diagnosis of unstable angina is essential, since it is associated with a high risk of adverse cardiac events; more than in the case of stable angina. About 35% of unstable angina patients undergo revascularisation during the index visit. Importantly, missed unstable angina patients that are sent home, often return for revascularisation within one year (-50%), or suffer myocardial infarction (8% in 30 days; MINERVA database Meander MC Amersfoort). As for stable angina, the diagnosis for unstable angina is similarly challenging. Troponin is negative per definition and the ECG is diagnostic in only 10-15% of cases. Hospitalisation is often required for determining serial cardiac troponin levels, non-invasive testing such as exercise ECG or imaging, or for an invasive coronary angiogram. No rapidly determinable and reliable diagnostic markers are available that positively identify the unstable angina patient, or that will on the other hand, exclude unstable angina.

### Blood biomarkers

Clearly, as outlined above, the establishment of a timely identification of a subject suffering from, or being at risk of suffering from, stable angina and unstable angina is important to allow adequate treatment. An accurate early diagnosis may take away the symptoms and improve the prognosis of the patients. In addition, ruling out both diseases prevents unnecessary referrals and hospital admissions for time consuming non-invasive and invasive diagnostic testing, thereby significantly decreasing the health care burden. A simple and rapid test for all patients presenting with symptoms suggestive of stable and unstable angina is highly desired. It has been recognized in the art that a blood test is the most convenient, since it is a low risk diagnostic tool and easily accessible in both primary and secondary care.

Many proteins have been investigated in relation to stable and unstable angina. The inflammatory concept of atherosclerosis led many investigators to concentrate on inflammatory mediators such as hsCRP, GDF-15, neopterin, IL-6, IL-10, IL-17, MPO, procalcitonin, Fetuin A, Lp-PLA2, and MMPs/TIMPs. Although these markers have some prognostic value, none of them appear to have sufficient diagnostic power to discriminate patients with stable and unstable angina (Tsaknis et al. Clinical usefulness of novel serum and imaging biomarkers in risk stratification of patients with stable angina. Dis Markers 2014:831364).

Also non-protein markers have been investigated. In a small pilot study involving 53 patients, three micro RNAs (miRNAs) appeared to have the potential to discriminate patients with stable angina from controls. MiRNAs are small non-coding RNAs that regulate complex biological processes. The miRNAs that were found were miR-1, miR-126, miR-483-5p, having an Area Under the Curve (AUC) of 0.91, 0.92 and 0.85 respectively (D'Alessandra et al. Diagnostic potential of plasmatic MicroRNA signatures in stable and unstable angina. PLoS One. 2013 Nov 15:8). However, these miRNA have thus far not been validated. In the same pilot study, three potential biomarkers for unstable angina were also found: miR-1, miR-126 and miR-133a, with AUC's of 0.92, 0.87 0.91 respectively. In contrast however, these miRNAs were not identified as potential biomarkers for unstable angina in other studies. In yet another investigation, a miRNA panel (consisting of miR-132, miR-150 and miR-186) showed the highest discriminating power with an AUC of 0.91 (Zeller et al. Assessment of microRNAs in patients with unstable angina pectoris. Eur Heart J. 2014 Aug 14;35(31):2106-14). It is evident that miRNAs could potentially be used as biomarkers for stable and unstable angina, but additional studies are required to validate their potential and to address inconstancies between the different studies. Next to this, detection of miRNA is technically challenging requiring cDNA synthesis and qPCR which limits their application in an acute setting (such as with unstable angina) or in GP settings.

It is concluded that there remains an urgent need for alternative circulating biomarkers to identify subjects suffering from, or being at risk of suffering from, an ischemic heart disease and to discriminate between life-threatening events such as stable and unstable angina of patients with chest pain or equivalent symptoms on the one hand, and milder events in people that also experience similar symptoms but do not suffer from such an ischemic heart disease, on the other hand.

### SUMMARY OF THE INVENTION

The present invention relates to a method for identifying a subject suffering from, or being at risk of suffering from, an ischemic heart disease as set out in the appended set of claims, wherein the ischemic heart disease is stable angina. The method comprises the steps of:
a) obtaining one or more of the plasma fractions selected from the group consisting of the Low-Density Lipoprotein (LDL) fraction, and the High-Density Lipoprotein (HDL) fraction from a plasma sample of said subject;
b) determining one or more values, wherein each of the one or more values:
   - is a value derived from a concentration of a protein marker in one of said plasma fractions, wherein the protein marker is selected from the group consisting of SerpinC1, Cystatin C, CD14, SerpinF2 and SerpinG1, wherein at least one of the one or more values is derived from a concentration of SerpinC1, or
   - is a value which is a ratio of two values derived from the concentrations of a single protein marker in two different ones of said plasma fractions, wherein the protein marker is selected from the group consisting of SerpinC1, Cystatin C, CD14, SerpinF2 and SerpinG1, or
   - is a value which is a combination of a value derived from a concentration of a protein marker associated with the extracellular vesicles in one of said plasma fractions, wherein the protein marker is selected from the group consisting of SerpinC1, Cystatin C, CD14, SerpinF2 and SerpinG1, and a value which is a ratio of two values derived from the concentrations of a single protein marker associated with the extracellular vesicles in two different ones of said plasma fractions, wherein the protein marker is selected from the group consisting of SerpinC1, Cystatin C, CD14, SerpinF2 and SerpinG 1,
c) and wherein the one or more values are selected such that the area under the curve (AUC) differs from the diagonal reference line of 0.5 with a p-value of 0.05 or less, as determined by Receiver Operating Characteristic (ROC) plot analysis of the combination of said one or more values based on a suitable group of definitive subjects and group of reference subjects, performing a comparison of the one or more values as determined in step b) with one or more corresponding reference values, which has been derived in the same way from the concentration of the same one or more protein marker in corresponding plasma fractions as determined in a group of reference subjects not suffering from ischemic heart disease, wherein a statistically significant difference between the one or more values determined in step b) and the one or more corresponding reference values is indicative of the subject suffering, or being at risk of suffering from,stable angina.

By this method it is for the first time possible to identify subjects suffering from stable angina, from a blood sample with a high level of statistical significance.

In a preferred embodiment, at least one of the one or more values in step b) is selected from the group consisting of values derived from the concentration of:
- SerpinC1 in the HDL plasma fraction,
   and
- CD14 in the HDL plasma fraction,
and/or wherein at least one of the one or more values is a ratio of two values derived from concentrations of a single protein marker in two different ones of said plasma fractions, selected from the group consisting of:
- the concentration of SerpinC1 in the LDL plasma fraction over the concentration in the HDL plasma fraction,
- the concentration of CD14 in the LDL plasma fraction over the concentration in the HDL plasma fraction,
- the concentration of Cystatin C in the LDL plasma fraction over the concentration in the HDL plasma fraction,
- the concentration of SerpinF2 in the LDL plasma fraction over the concentration in the HDL plasma fraction.

This embodiment of the invention provides for the first time a method to identify subjects suffering from, or being at risk of suffering from, stable angina from a blood sample with a high level of statistical significance.

Described herein but not part of the invention is a kit comprising means for performing the method according to the invention.

### BRIEF DESCRIPTION OF THE DRAWINGS

These and other aspects of the invention are apparent from and will be elucidated with reference to the embodiments described hereinafter.

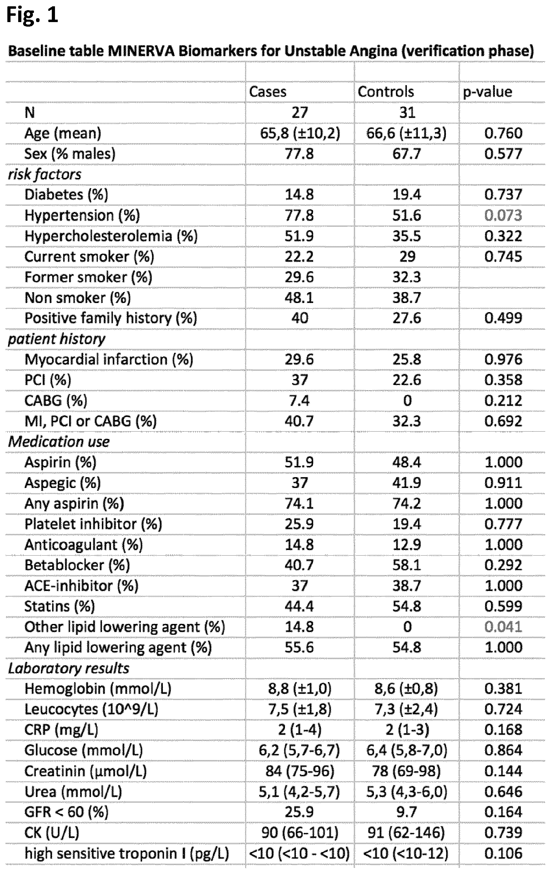

**Figure 1** (not according to the invention and for illustration purposes only) is a table showing the baseline of 30 unstable angina cases and 30 matched controls.

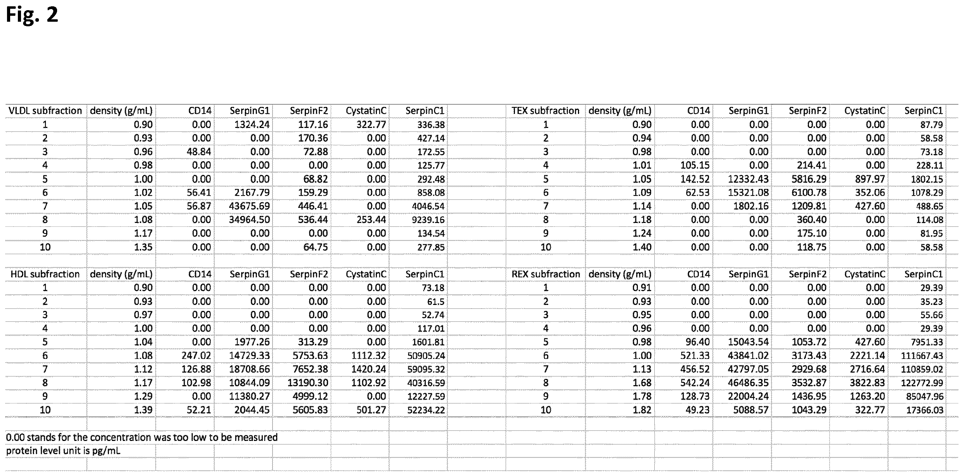

**Figure 2** is a table showing the measurement of CD14, SerpinG1, SerpinF2, Cystatin C and SerpinC1 levels in each of the ten density gradient fractions per plasma sub-fraction.

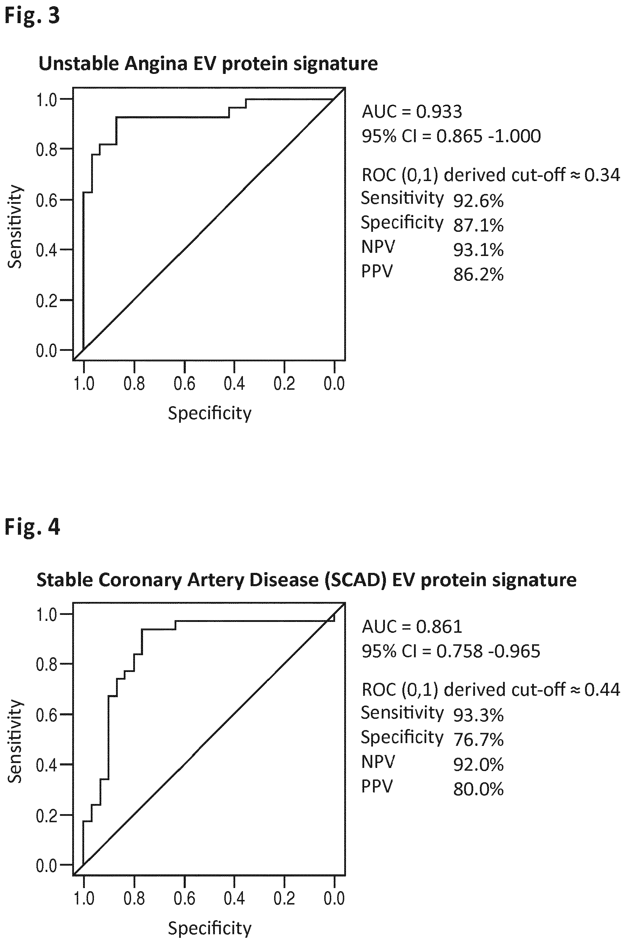

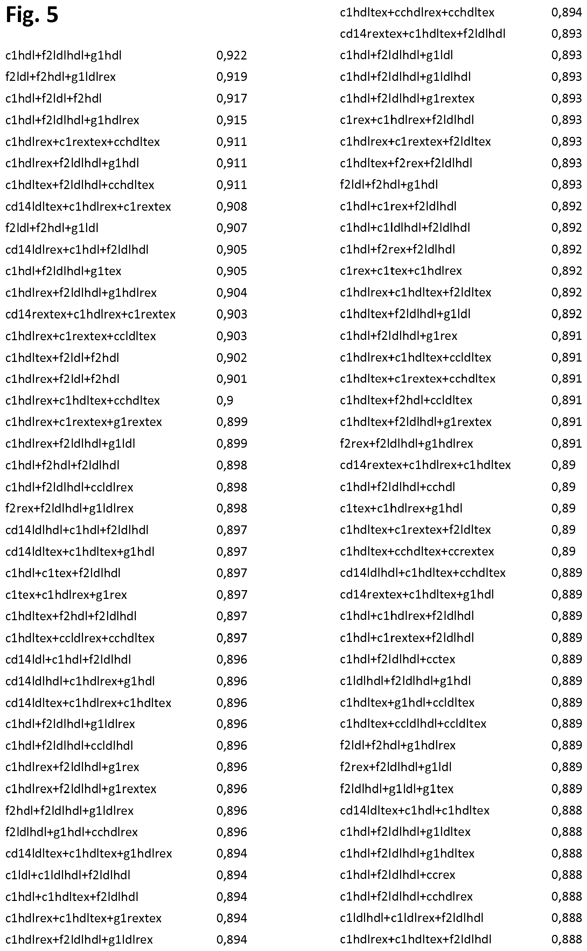

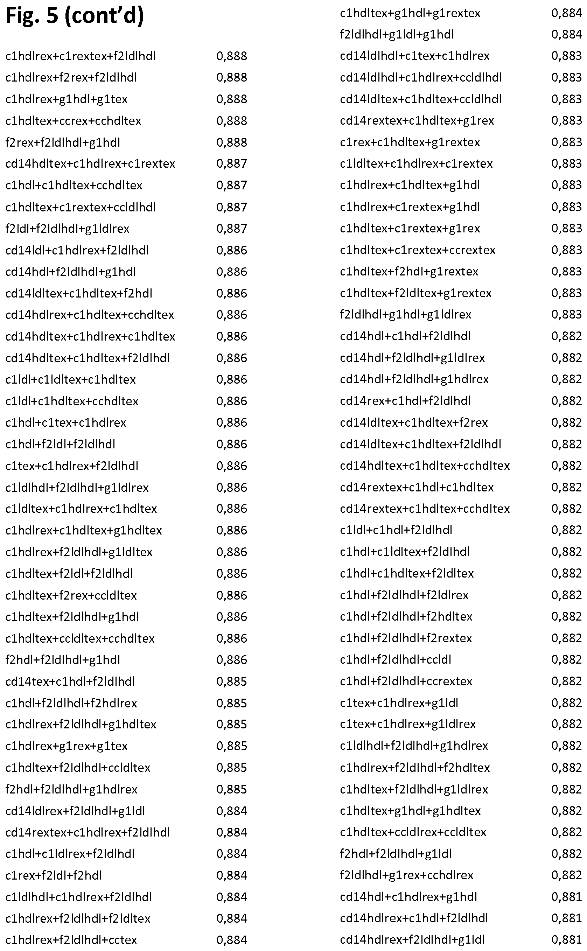

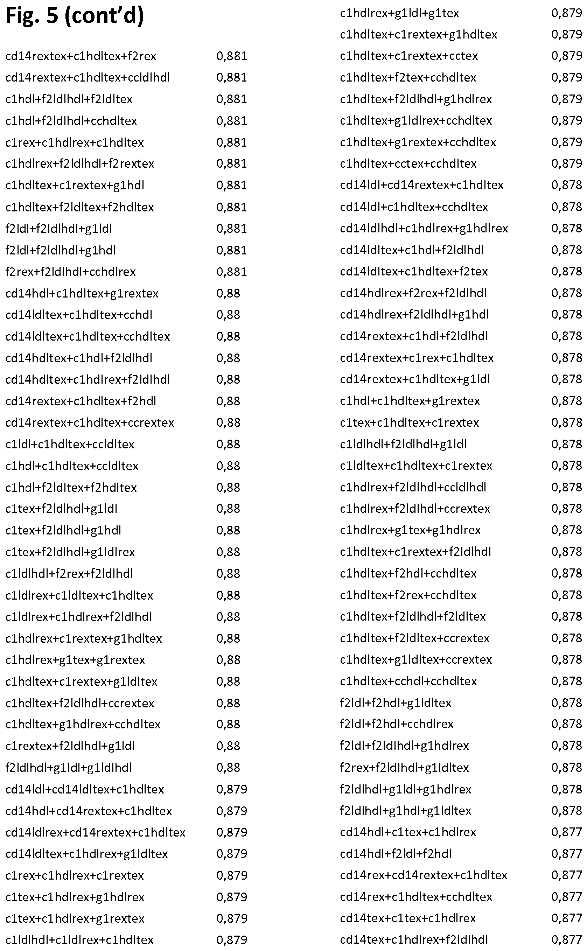

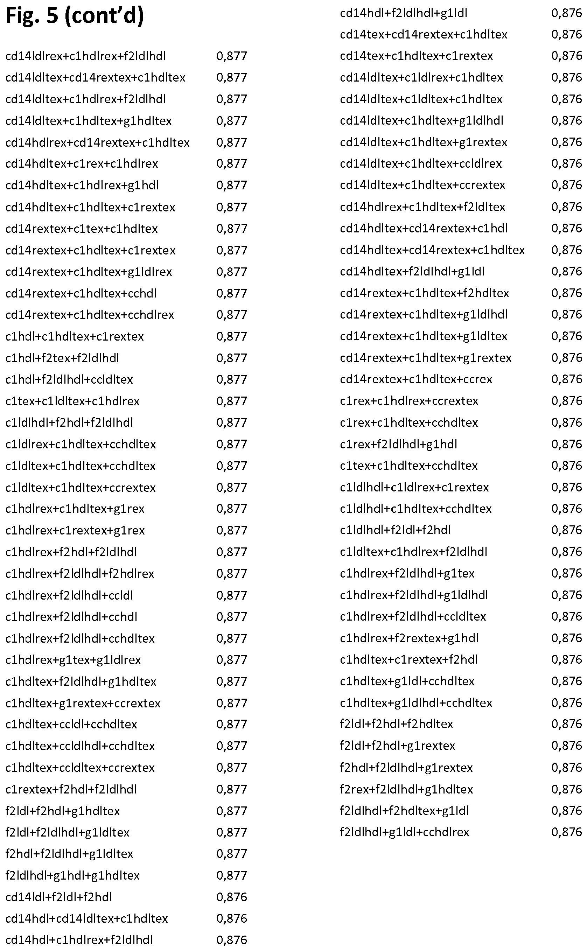

**Figure 3** (not according to the invention and for illustration purposes only) displays the Receiver Operating Characteristic (ROC) plot of the identification of unstable angina in an Emergency Department cohort of chest pain patients of 30 definitive unstable angina and 30 matched controls using the combination of markers consists of SerpinC1-HDL + CD14-TEX + SerpinC1-LDL. **Figure 4** shows a ROC plot of the identification of stable angina (here referred to as stable coronary artery disease (SCAD)) in a cohort of suspected symptomatic coronary artery disease of 30 definitive cases with stable angina and 30 matched controls using the combination of markers consists of SerpinC1-HDL + SerpinG1-TEX + SerpinC1-REX. **Figure 5** shows the highest scoring combinations of three marker/fraction and/or marker/ratio in the Myomarker cohort (two columns per page, each column showing the marker combination on the left and its AUC value on the right). Abbreviations: c1=SerpinC1, g1=SerpinG1, f2=SerpinF2, cc=Cystatin C, cd14=CD14, Idl=Low-Density lipoprotein, hdl=High-Density Lipoprotein, rex=remaining fraction, tex=total fraction. Whenever the abbreviation of two plasma fractions are mentioned in connection with a protein marker, it means that the ratio is taken between the concentration (or a value derived therefrom) in these two plasma fractions. By way of example, "c1ldlhdl" means the ratio of a value derived from the concentration of SerpinC1 in fraction LDL over HDL. In total the list contained 19,600 combinations, only the combinations with an AUC of 0.876 or higher are shown.

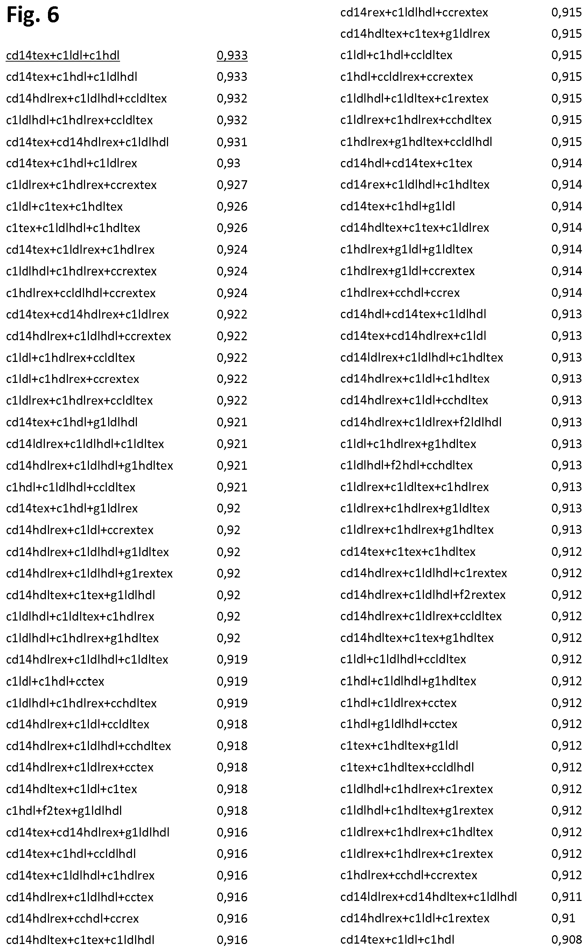

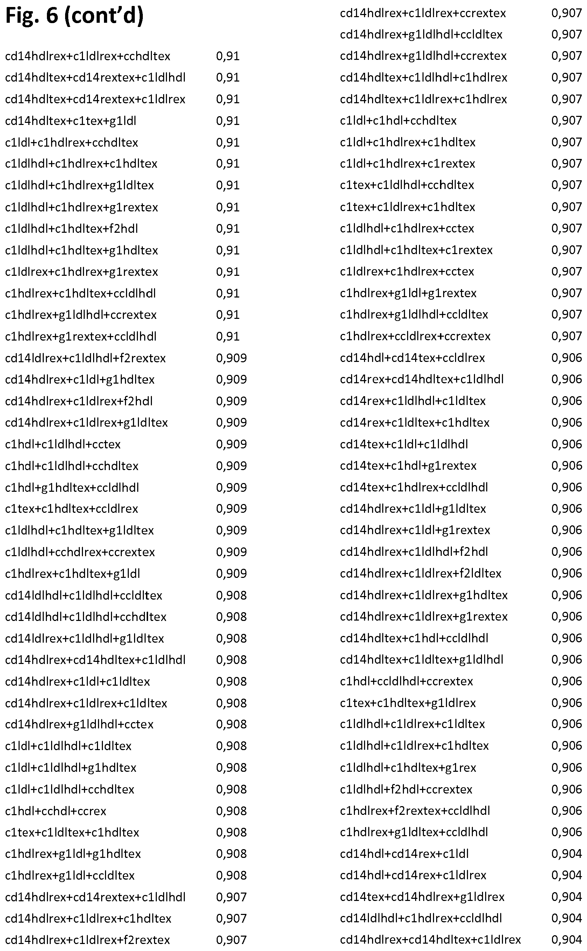

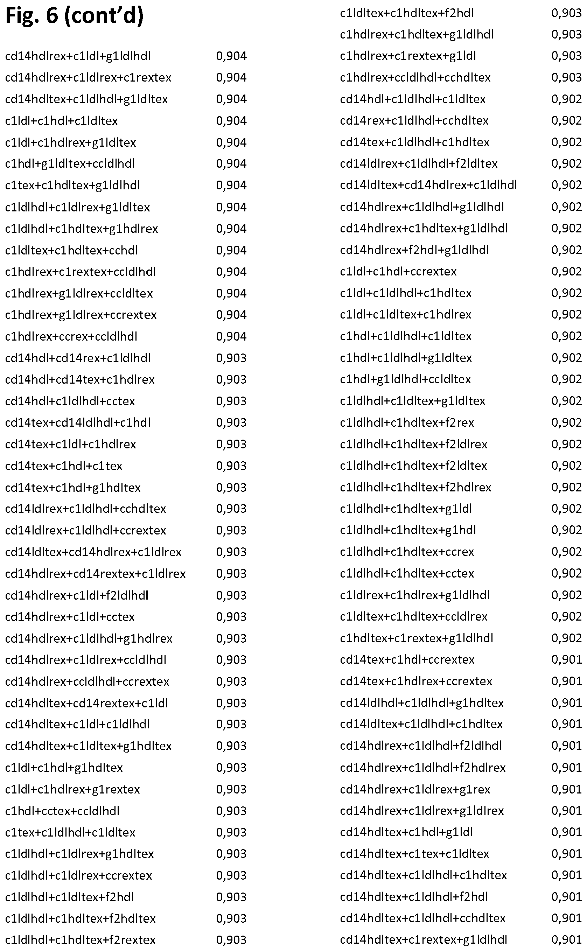

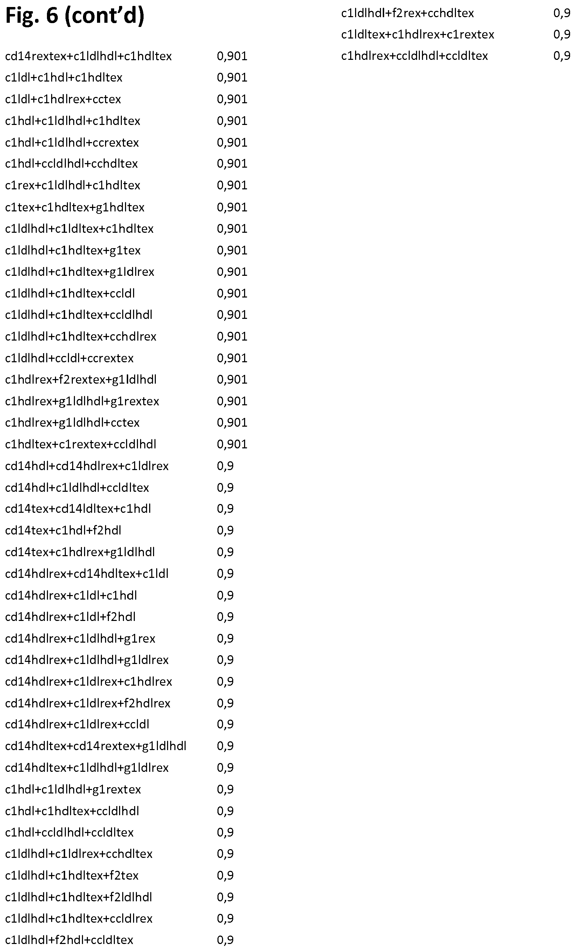

**Figure** 6 (not according to the invention and for illustration purposes only) shows the highest scoring combinations of three marker/fraction and/or marker ratio pairs in the Minerva cohort (two columns per page, each column showing the marker combination on the left and its AUC value on the right). Abbreviations are as in Figure 5 and so is the nomenclature for when two plasma fractions are mentioned in connection with a protein marker. In total the list contained 19,600 combinations, only the combinations with an AUC of 0.9 or higher are shown. The best scoring combination of marker/fraction pairs (i.e. comprising no marker/ratio pairs) SerpinC1-HDL + CD14-TEX + SerpinC1-LDL (AUC 0.933) depicted in Fig. 3 is underlined.

### DETAILED DESCRIPTION OF EMBODIMENTS

The present invention relates to a method for identifying a subject suffering from, or being at risk of suffering from, stable angina from a blood sample based on the concentration (or a value derived from the concentration) of one or more identified protein markers in one or more blood plasma fractions or based on the ratio of concentration (or values derived from the concentration) of a given protein marker in two different ones of said plasma fractions and comparing it to the corresponding concentration and/or ratio as determined in a group of reference subjects not suffering from ischemic heart disease, wherein a statistically significant difference between the concentration and/or ratio determined for the subject in question and the corresponding concentration and/or ratio of the reference group is indicative of the subject suffering, or being at risk of suffering, fromstable angina. In the context of the present invention, "concentration" of a given protein marker in a given plasma sub-fraction can also mean a "value derived from the concentration" of the protein marker in the plasma sub-fraction in question, wherein a value derived from the concentration is a value that directly correlates with the concentration of the protein marker. In one embodiment, the present invention relates to a method for identifying a subject suffering from, or being at risk of suffering from, stable angina, said method comprising the steps of:
a) obtaining one or more of the plasma fractions selected from the group consisting of the Low-Density Lipoprotein (LDL) fraction, the High-Density Lipoprotein (HDL) fraction, the remaining (REX) fraction and the total (TEX) fraction from a plasma sample of said subject;
b) determining one or more values, wherein each of the one or more values:
   - is derived from a concentration of a protein marker in one of said plasma fractions, wherein the protein marker is selected from the group consisting of SerpinC1, Cystatin C, CD14, SerpinF2 and SerpinG1, wherein at least one of the one or more values is derived from a concentration of SerpinC1, or
   - is a ratio of two values derived from the concentrations of a single protein marker in two different ones of said plasma fractions, wherein the protein marker is selected from the group consisting of SerpinC1, Cystatin C, CD14, SerpinF2 and SerpinG1, or
   - is a value which is a combination of a value derived from a concentration of a protein marker associated with the extracellular vesicles in one of said plasma fractions, wherein the protein marker is selected from the group consisting of SerpinC1, Cystatin C, CD14, SerpinF2 and SerpinG1, and a ratio of two values derived from the concentrations of a single protein marker associated with the extracellular vesicles in two different ones of said plasma fractions, wherein the protein marker is selected from the group consisting of SerpinC1, Cystatin C, CD14, SerpinF2 and SerpinG1,
c) performing a comparison of the one or more values as determined in step b) with one or more corresponding reference values, which has been derived in the same way from the concentration of the same one or more protein marker in corresponding plasma fractions as determined in a group of reference subjects not suffering fromstable angina, wherein a statistically significant difference between the one or more values determined in step b) and the one or more corresponding reference values is indicative of the subject suffering, or being at risk of suffering, fromstable angina.

By this method it is for the first time possible to identify subjects suffering from stable angina, from a blood sample with a statistical relevance characterized by that the one or more values in step b) are selected such that the area under the curve (AUC) differs from the diagonal reference line of 0.5 with a p-value of 0.05 or less, as determined by Receiver Operating Characteristic (ROC) plot analysis of the combination of said one or more values based on a suitable group of definitive subjects and group of reference subjects. Preferably, the statistical relevance of the method according to the invention is characterized by that the one or more values in step b) are selected such that the area under the curve (AUC) is 0.8 or more, such as 0.85 or more, 0.9 or more or 0.95 or more and the p-value is 0.05 or less, as determined by Receiver Operating Characteristic (ROC) plot analysis of the combination of said one or more values based on a suitable group of definitive subjects and group of reference subjects. Even more preferably, the statistical relevance of the method according to the invention is characterized by resulting in a negative predictive value and/or a positive predictive value is 0.8 or more, such as 0.85 or more, 0.9 or more or 0.95 or more. In one embodiment, the protein marker is SerpinC1. The method according to the present invention is an in vitro method.

In the context of the present invention, the subject may be any mammal but is preferably a human subject and even more preferably a human patient, such as a human patient having chest pain. In the context of the present invention, the group of reference subjects is of the same origin as the subject itself, and accordingly, if the subject is a human, the group of reference subjects are also human. In one embodiment of the present invention, the group of reference subjects have the same clinical signs and symptoms as the subject itself but are not suffering from ischemic heart disease.

The plasma fractions used in the present invention can be obtained as follows:
Using dextrane sulphate and Mn, chylomicrons, Very Low-Density Lipoprotein (VLDL) and Low-Density Lipoprotein (LDL) are precipitated in the first fractionation step. Hereinafter, the plasma fraction obtained in this first fractionation step is called "LDL". In the second step High-Density Lipoprotein (HDL) is precipitated. Sub-fractionation of the LDL and HDL fractions from plasma is known in the art. The remaining sub-fraction is referred to as (REX), is almost completely depleted of lipoprotein particles. The inventors of the present invention have investigated the protein content of plasma extracellular vesicles present in three different blood plasma sub-fractions: LDL, HDL and the remaining plasma subtraction REX. Besides that, the inventors have assessed the protein patterns associated with extracellular vesicles in unfractionated (total) plasma (referred to as TEX). The TEX fraction can be obtained by using Exoquick precipitation buffer sold by SBI or Xtractt buffer from Cavadis B.V. in accordance with manufacturers' instructions. In the context of the present invention, the term "plasma fraction" refers to the LDL, HDL, REX, and TEX plasma fractions. The terms "plasma fraction" and "plasma sub-fraction" are used interchangeably herein.

Plasma extracellular vesicles are bilayer lipid membrane vesicles including exosomes, microvesicles and microparticles (Colombo et al. Biogenesis, secretion, and intercellular interactions of exosomes and other extracellular vesicles. Ann Rev Cell Dev Biol 2014:255-289). Exosomes are synthesized in the multivesicular endosome, while microvesicles are formed by the plasma membrane. Once secreted in the plasma these extracellular vesicles can no longer be distinguished from each other. This is why exosomes are often called microvesicles and microvesicles are often referred to as exosomes. For the sake of clarity, extracellular vesicles as used herein refer to all such extracellular bilayer lipid membrane vesicles present in the sub-fractions of the plasma, as outlined further below.

Extracellular vesicles play an important role in intercellular communication and contain or are associated with proteins, miRNAs, and mRNA from the cell of origin, reflecting their physiological or pathological status. It is known that distinct bilayer membrane extracellular plasma vesicles co-fractionate with monolayer LDL. Other bilayer membrane extracellular vesicles (with a different content) co-fractionate with HDL (Zhang et al. Circulating TNFR1 exosome-like vesicles partition with the LDL fraction of human plasma. Biochem Biophys Res Comm 2008:579-584). This allows separation of distinct plasma extracellular vesicle sub-fractions via sequential LDL and HDL isolation. Through this, the inventors were able to identify subpopulations of extracellular vesicles, each with their own particular protein content and potentially (patho)-physiological pathways associated therewith. Plasma extracellular vesicles have been recognized in the art as having potential value in relation to cardiovascular disease (Wang et al. Plasma extracellular vesicle protein content for diagnosis and prognosis of global cardiovascular disease. Neth Heart J 2013:467-471).

In the context of the present invention, protein marker SerpinC1 is identified as *UniProtKB* - *P01008 (ANT3_HUMAN*), protein marker Cystatin C is identified as *UniProtKB* - *P01034 (CYTC_HUMAN),* protein marker CD14 is identified as *UniProtKB* - *P08571 (CD14_HUMAN*), protein marker SerpinF2 is identified as *UniProtKB* - *P08697 (A2AP_HUMAN*) and protein marker SerpinG1 is identified as *UniProtKB* - *P05155* (*IC1_HUMAN*), when the subject is a human, such as a human patient. The skilled person will understand, that if the subject is another mammal than human, the protein markers to be used in accordance with the invention, will be the corresponding proteins in the mammal in question.

In the context of the present invention, a value derived from the concentration of a given protein marker in a given plasma fraction is also called a value of a "marker/fraction pair". For convenience, a marker/fraction pair is sometimes abbreviated herein by [name of the marker]-[name of the plasma fraction]. For example, the protein marker SerpinC1 determined in plasma fraction LDL interchangeably is also referred to by "SerpinC1 in LDL", "SerpinC1-LDL" or "C1-LDL". Likewise, a value which is a ratio of two values derived from the concentrations of a given protein marker in two different plasma fractions is called a value of a "marker/ratio pair". For convenience, a marker/ratio pair is sometimes abbreviated herein by [name of the marker]-[name of plasma fraction 1]/[name of plasma fraction 2]. For example, the protein marker SerpinC1 determined in plasma fraction LDL and HDL and for which the ratio of the values derived from the concentrations in LDL over HDL is used, is also referred to by "ratio of SerpinC1 in LDL over HDL", "SerpinC1-LDUHDL" or "C1-LDL/HDL". The value derived from the concentration of a given protein marker in a given plasma fraction can be determined in any way known to a person skilled in the art. In one embodiment of the present invention, the determination of the one or more values in step b) is performed by an immunoassay using antibodies specific to the one or more protein markers in question. The immunoassay can suitably be a beads-based immunoassay, wherein the beads are conjugated with the selected antibodies to synthesize the bead-capture antibody complex. The bead-capture antibody complex is then incubated with the samples and subsequently with biotinylated antibodies to detect the captured protein by reaction with streptavidin subsequent and quantification.

The one or more values of marker/fraction and/or marker/ratio pairs selected in step b), are selected based on their individual or combined statistical relevance for identification of a subject suffering from, or at risk of suffering from stable angina. The statistical relevance can be determined in any way known to the person skilled in the art. Logistic regression analysis is often used to predict a binary outcome (yes or no). In medical research it is often used to predict if a patient has a certain disease, for example diabetes (yes or no) by modelling observed characteristics of the patients e.g. sex, age, weight and systolic blood pressure. In the diabetes example a result from logistic regression could be that a 10-year increase in age gives a 20% higher odds of having diabetes. By combining more probabilities, based on more than one patient characteristic in one prediction model, one can even more accurately predict if a patient has diabetes or not. Next to using patient characteristics like sex and age, logistic regression can also be performed with biomarker levels associated with extracellular vesicles as potential predictors. A set of biomarkers can be combined in one model and used to estimate the probability of a disease. The performance of a logistic regression model can be visualized in a Receiver Operating Characteristic (ROC) plot. The higher the Area Under the Curve (AUC), the better the performance of the model. An AUC of 0.5 corresponds to a 50% chance of the disease in question (flipping a coin). Here, the inventors used logistic regression and ROC plot analysis in order to evaluate whether the diagnosis of stable coronary artery disease could be improved based on the five extracellular vesicle proteins in 4 different plasma sub-fractions and whether such be better than by random chance. The present inventors determined the statistical relevance of the different individual marker/fraction pairs and marker/ratio pairs (tables 1 and 3) and of combinations thereof (tables 2 and 4 and figures 5 and 6) by ROC plot analysis. The ROC plot analysis involves:
- analyzing Differences in baseline-characteristics using Chi-square test for categorical variables
- performing T-tests for normally distributed continuous variables and Mann-Whitney-U-tests for continuous variables that were not normally distributed
- converting the one or more values derived from the concentration in a given plasma fraction or ratio of concentrations in two different plasma fractions into standard deviation units, or the z-score, by using the observed value minus the mean value, divided by the standard deviation, and
- Performing Receiver-operating characteristic (ROC) analysis (ROC plot) to determine the area under the curve (AUC) and optimal calculated cutoff for the negative predictive value (NPV) and/or the positive predictive value (PPV).

This statistical analysis can be performed by a any suitable software, for example by SPSS^{®} (IBM^{®}, Version 22) and Rstudio using R software for statistical computing version 3.1.2. The skilled person will understand, that where more than one value of marker/fraction and/or marker/ratio pairs are used, the AUC and calculated cut-off value obtained from the ROC plot analysis relate to the combination of said values.

The one or more values in step b) are selected such that the area under the curve (AUC) differs from the diagonal reference line of 0.5 with a p-value of 0.05 or less, as determined by Receiver Operating Characteristic (ROC) plot analysis of the combination of said one or more values based on a suitable group of definitive subjects and group of reference subjects. In a further embodiment, the one or more values in step b) are selected such that the area under the curve (AUC) is 0.8 or more, such as 0.85 or more, 0.9 or more or 0.95 or more, and the p-value is 0.05 or less, as determined by Receiver Operating Characteristic (ROC) plot analysis of the combination of said one or more values based on a suitable group of definitive subjects and group of reference subjects. In yet a further embodiment, the negative predictive value and/or the positive predictive value is 0.8 or more, such as 0.85 or more, 0.9 or more or 0.95 or more, for the selected one or more values in step b), when using the optimal cut-off value as determined by Receiver Operating Characteristic (ROC) plot analysis of the combination of said one or more values based on a suitable group of definitive subjects and group of reference subjects. In the context of the present invention the term "group of definitive subjects" means a group of subjects that has been verified to suffer from stable angina by other means than by the method of the present invention. This group could also be called the positive control group. The term "group of reference subjects" as used herein, is a group of subjects that has been verified not to suffer from stable angina by other means than by the method of the present invention. This group could also be called the negative control group. In one embodiment of the present invention, the group of reference subjects have the same clinical signs and symptoms as the group of definitive cases but are not suffering fromstable angina. In a further embodiment, the suitable group of definitive subjects and group of reference subjects is a suitable cohort, such as the Myomarker cohort. In the context of the present invention, the Myomarker cohort comprises a group of definitive subjects, which have been diagnosed to suffer from stable angina and a group of reference subjects, which have the same clinical signs and symptoms as the group of definitive cases, but which are not suffering from stable angina, wherein the assessment of whether or not a subject falls in the group of definitive cases or in the group of reference subjects is performed by other means than by the method of the present invention. The comparison performed in step c) of the method according to the present invention can therefore alternatively be implemented as performing a comparison using a model and a cut-off value as determined by Receiver Operating Characteristic (ROC) plot analysis of the combination of said one or more values based on a suitable group of definitive subjects and group of reference subjects, wherein the outcome of said model is indicative of the subject suffering from, or being at risk of suffering from stable angina.

In a further embodiment, the present invention relates to a method for determining one or more values derived from a concentration of a protein marker in a plasma fraction or from a ratio of concentrations of a single protein marker in two different plasma fractions, said method comprising the steps of:
a) obtaining one or more of the plasma fractions selected from the group consisting of the Low-Density Lipoprotein (LDL) fraction and the High-Density Lipoprotein (HDL) fraction from a plasma sample of said subject;
b) determining one or more values, wherein each of the one or more values:
   - is derived from a concentration of a protein marker in one of said plasma fractions, wherein the protein marker is selected from the group consisting of SerpinC1, Cystatin C, CD14, SerpinF2 and SerpinG1, and/or
   - is a ratio of two values derived from the concentrations of a single protein marker in two different ones of said plasma fractions, wherein the protein marker is selected from the group consisting of SerpinC1, Cystatin C, CD14, SerpinF2 and SerpinG1.

The method may further comprise performing a comparison of the one or more values as determined in step b) with one or more corresponding reference values, which has been derived in the same way from the concentration of the same one or more protein marker in corresponding plasma fractions as determined in a group of reference subjects not suffering fromstable angina. When there is a statistically significant difference between the one or more values determined in step b) and the one or more corresponding reference values, it is indicative of the subject suffering, or being at risk of suffering, from stable angina.

The inventors of the present invention showed earlier that the protein content of all plasma extracellular vesicles at 6 hours after the onset of symptoms appeared to be associated with the diagnosis of non ST-elevation myocardial infarction (De Hoog et al. Serum extracellular vesicle protein levels are associated with acute coronary syndrome. Eur Heart J Acute Cardiovasc Care 2013. 2(1):53-60) establishing that the content of unfractionated (total) plasma extracellular vesicles is rapidly changing after ischemic heart disease. In the current invention, a different sample of plasma sub-fractions was used, in which the inventors assessed the extracellular vesicle protein content. Plasma samples were derived from patients with chest pain at the emergency department that were finally diagnosed to suffer from unstable angina and from individuals that also entered the emergency department with chest pain but that did not have ischemic heart disease (serving as controls). The inventors performed proteomics on the extracellular vesicle sub-fractions from both groups. Proteins were characterized in the extracellular vesicles present in the LDL, HDL, REX and TEX extracellular plasma sub-fractions of in total 30 established unstable angina chest pain patients and in a total of 30 of the age-, sex-, risk-, history- and medication-matched control chest pain emergency department patients (without unstable angina).

Although unstable angina is thought to be a thrombotic event in contrast to stable angina, there is mechanistic overlap between the two clinical syndromes: there is coronary artery disease and myocardial ischemia (reduction in blood flow) in both disorders. For this, the inventors performed similar immuno bead assay experiments on blood (plasma) samples from 30 established stable coronary artery disease chest pain patients versus 30 age-, sex-, risk-, history-, and medication-matched control chest pain patients in the extracellular plasma sub-fractions LDL, HDL, REX, and TEX.

In a one embodiment, the present invention relates to a method for identifying a subject suffering from, or being at risk of suffering from, stable angina, said method comprising the steps of:
a) obtaining one or more of the plasma fractions selected from the group consisting of the Low-Density Lipoprotein (LDL) fraction and the High-Density Lipoprotein (HDL) fraction from a plasma sample of said subject;
b) determining at least one of the one or more values is selected from the group consisting of values derived from the concentration of:
   - SerpinC1 in the HDL plasma fraction,
      and
   - CD14 in the HDL plasma fraction,
   and/or wherein at least one of the one or more values is a ratio of two values derived from concentrations of a single protein marker in two different ones of said plasma fractions, selected from the group consisting of:
   - the concentration of SerpinC1 in the LDL plasma fraction over the concentration in the HDL plasma fraction,
   - the concentration of CD14 in the LDL plasma fraction over the concentration in the HDL plasma fraction,
   - the concentration of Cystatin C in the LDL plasma fraction over the concentration in the HDL plasma fraction,
   - the concentration of SerpinF2 in the LDL plasma fraction over the concentration in the HDL plasma fraction, and
c) performing a comparison of the one or more values as determined in step b) with one or more corresponding reference values, which has been derived in the same way from the concentration of the same one or more protein marker in corresponding plasma fractions as determined in a group of reference subjects not suffering from stable angina, wherein a statistically significant difference between the one or more values determined in step b) and the one or more corresponding reference values is indicative of the subject suffering, or being at risk of suffering, from stable angina.

The present inventors have found that these values of marker/fraction pairs and marker/ratio pairs provide a statistically relevant identification of subjects suffering from stable angina on their own (see table 3). Accordingly, combination with further marker/fraction and/or marker/ratio values is not required but will in many cases improve the certainty by which a subject is correctly identified as suffering from stable angina. Accordingly, the method according to this embodiment of the invention provides for the first time a method to identify subjects suffering from stable angina from a blood sample with a statistical relevance characterized by that the one or more values in step b) are selected such that the area under the curve (AUC) differs from the diagonal reference line of 0.5 with a p-value of 0.05 or less, as determined by Receiver Operating Characteristic (ROC) plot analysis of the combination of said one or more values based on a suitable group of definitive subjects and group of reference subjects. Preferably, the statistical relevance of the method provided in this embodiment of the invention is characterized by that the one or more values in step b) are selected such that the area under the curve (AUC) is 0.8 or more, such as 0.85 or more, 0.9 or more or 0.95 or more and the p-value is 0.05 or less, as determined by Receiver Operating Characteristic (ROC) plot analysis of the combination of said one or more values based on a suitable group of definitive subjects and group of reference subjects. Even more preferably, the statistical relevance of this embodiment of the invention is characterized by resulting in a negative predictive value and/or a positive predictive value is 0.8 or more, such as 0.85 or more, 0.9 or more or 0.95 or more.

The present inventors have found that the marker/fraction value of SerpinC1 in the HDL and the marker/ratio values of SerpinC1 in HDL/REX, SerpinC1 in HDL/TEX, CD14 in HDL/TEX and SerpinF2 in LDL/HDL all individually provide an AUC value of 0.8 or more and a p-value of 0.05 or less in a ROC plot performed as described above. Accordingly, combination with further marker/fraction and/or marker/ratio values is not required but will in many cases improve the certainty by which a subject is correctly identified as suffering from stable angina. A preferred embodiment of the present invention is therefore a method for identifying a subject suffering from, or being at risk of suffering from, stable angina, at least one of the one or more values is derived from the concentration of SerpinC1 in the HDL plasma fraction and/or at least one of the one or more values is a ratio of the concentration of SerpinF2 in the LDL plasma fraction over the concentration in the HDL plasma fraction.

Table1 in example 4 shows the marker/fraction and marker/ratio pairs which give the most statistically relevant identification of stable angina. The F2-LDUHDL marker/ratio pair has an AUC value of above 0.8 for identification of stable angina on its own (without combination with a further marker/fraction or marker/ratio value). The marker/ratio pairs C1-REX/TEX, F2-LDUREX, F2-HDL/TEX and G1-REX/TEX and the C1-TEX marker/fraction pair are shown in table 1 as being among the most significant single marker/fraction and marker/ratio pairs for identification of stable angina,Accordingly, in a preferred embodiment of the method for identifying a subject suffering from, or being at risk of suffering from, stable angina, wherein at least one of the one or more values is a ratio of two values derived from concentrations of a single protein marker in two different ones of said plasma fractions, selected from the group consisting of: the concentration of SerpinF2 in the LDL plasma fraction over the concentration in the HDL plasma fraction.

### Combination of marker/fraction and/or marker/ratio pairs.

Table 2 in example 4 shows the statistically most relevant combinations of two marker/fraction and/or marker/ratio pairs for the identification of stable angina in the Myomarker cohort. It follows from table 4, that the statistically most significant combination of two marker/fractions and/or marker/ratio pairs for identification of stable angina as determined in herein is the combination of SerpinC1-HDL and SerpinF2-LDL/HDL with an AUC of 0.881 as determined by ROC plot analysis as described in the above. With an optimal cut-off value based on the AUC, this combination of SerpinC1-HDL and SerpinF2-LDUHDL has a sensitivity of 0.786 (95% Cl 0.590-0.917), a specificity of 0.852 (95% Cl 0.633-0.958), a negative predictive value (NPV) of 0.793 (95% Cl 0.601-0.938) and a positive predictive value of 0.846 (95% Cl 0.652-0.943). The table in Fig. 5 shows the statistically most relevant combinations of three of marker/fraction and/or marker/ratio pairs in the Myomarker cohort. It follows from the table in Fig. 5, that the statistically most significant combination of three marker/fractions and/or marker/ratio pairs for identification of stable angina as determined in herein is the combination of SerpinC1-HDL + SerpinF2-LDUHDL + SerpinG1-HDL with an AUC of 0.922 as determined by ROC plot analysis as described in the above. With an optimal cut-off value based on the AUC, the sensitivity of these three markers was 0.857 (95% Cl 0.673-0.960) and specificity of 0.852 (95% Cl 0.663-0.958). The Negative Predictive Value (NPV) was 0.852 (95% Cl 0.664-0.958) and the Positive Predictive Value was 0.857 (95% Cl 0.672-0.960). The present inventors found that SerpinC1 is one of the better protein markers when using only one marker/fraction or marker/ratio pair. From table 1 it follows that the 3 best individual marker/fraction or marker ratio pairs are SerpinC1 with the ratio HDL/TEX and an AUC of 0.842 (95% Cl 0.741-0.944); SerpinC1 with the ratio HDL/REX and an AUC of 0.828 (95% Cl 0.718-0.938)) and SerpinC1 in the HDL fraction with an AUC of 0.812 (95% Cl 0.700-0.924) to discriminate between stable angina and matched controls. Selection of the best combination of markers and sub-fractions without the ratios of markers between the sub-fractions showed an AUC of 0.861, when not taking any marker/ratio pair into account. It was concluded that one of the more optimal combination (panel) to diagnose stable angina appeared to consist of SerpinC1-HDL + SerpinG1-TEX + SerpinC1-REX. With an optimal cut-off value based on the AUC, the sensitivity of these 3 markers was 93.3% and specificity of 76.7%. The NPV is 92 % and the Positive Predictive Value is 80 %. Hence, the inventors of the present invention have now also found a method and means to discriminate between patients that experience stable angina and should be further treated, from patients that also have chest pain, but that do not suffer from stable angina, by preferably applying a protein concentration determination of SerpinC1 in HDL, , and comparing the concentrations with those found in a control sample. It is concluded here also that a blood-based assay as outlined abovecan now determine whether patients suffer from stable angina or not.

In conclusion, the skilled person will understand, that whereas the marker/fraction and marker/ratio pairs identified as having a statistically significant predictive value for the identification of subjects suffering from, or at risk of suffering from, stable angina, the predictive value will increase if more than one, such as at least two or at least three of the identified marker/fraction or marker/ratio pairs are included in the methods according to the present invention. In one embodiment of the present invention, the method for identifying a subject suffering from, or being at risk of suffering from, stable angina, comprises that at least two values, such as at least three values, are determined in step b). In another embodiment of the present invention, the method for identifying a subject suffering from, or being at risk of suffering from, stable angina, comprises that at least one of the one or more values determined in step b) is a ratio of two values derived from concentrations of a single protein marker in two different ones of said plasma fractions, wherein the protein marker is selected from the group consisting of SerpinC1, Cystatin C, CD14, SerpinF2 and SerpinG1. In another embodiment at least two, such as at least three, values are determined in step b) and at least one of these values is a ratio of two values derived from concentrations of a single protein marker in two different ones of said plasma fractions. In another embodiment of the present invention, at least two values involving at least two different protein markers are determined in step b), which values each can be selected from a value of a marker/fraction pair or a marker/ratio pair. In yet a further embodiment of the present invention at least one of said protein markers is SerpinC1, meaning that either a marker/fraction pair or a marker/ratio pair involving SerpinC1 is used in the method for identifying a subject suffering from, or being at risk of suffering from, an ischemic heart disease, such as stable or unstable angina. When one of the protein markers used in the method according to the present invention is SerpinC1, and at least one further protein marker is used in combination herewith, said at least one further protein marker is suitably selected from the group consisting of Cystatin C, CD14, SerpinF2 and SerpinG1. Suitably, all selected protein markers are different.

### Kit

Disclosed herein but not part of the invention is a kit comprising means for performing the method according to the invention. In one embodiment, the kit comprises means for determining the concentration of said protein markers in said fractions. Said means may comprise any suitable means known in the art, such as an immune-bead based process as outlined herein. Further, the kit may comprise instructions and means for the fractionation of a plasma sample to enable the rapid fractionation and subsequent protein concentration determination as outlined herein. The kit may also comprise means for performing the comparison of step c) in the form of an algorithm with a suitable cut-off value. Preferably, the cut-off value is determined by Receiver Operating Characteristic (ROC) plot analysis of the combination of said one or more values based on a suitable group of definitive subjects and group of reference subjects.

Patients with suspected ischemic heart disease constitute an enormous health care burden. Rapid and straightforward diagnostic tools for stable angina are currently not available. The technology used in this invention to characterize the protein content of the extracellular vesicles of the different plasma sub-fractions and the subsequent immune-based detection of the indicated proteins in such fractions is suitable for automated settings and ideal for point of care applications and has major implications for the efficiency of the diagnostic process and clinical management. The diagnosis of stable angina with the test as indicated herein now enables timely and adequate subsequent treatment. It will have a significant impact on the prognosis of the patient. On the other hand, ruling out both diseases prevents unnecessary hospital referrals/admissions that are expensive, time consuming and sometimes contain risky additional testing.

### Further embodiments

The present invention relates to a method for diagnosing a human patient as suffering from stable angina, said method comprising: obtaining a Low-Density Lipoprotein (LDL) and a High-Density Lipoprotein (HDL) of a plasma sample from said patient; determining the concentration of a protein marker in said LDL and HDL fractions; determining the concentration of said protein marker in reference fractions from a human subject not suffering fromstable angina; and determining whether said patient suffers from stable angina based on the difference in the concentrations determined in the fractions of said patient and said subject not suffering from stable angina, wherein said protein marker is selected from the group consisting of: human SerpinC1, SerpinG1, CD14 and SerpinF2, whereby the concentration of SerpinC1 is determined. In another aspect of the invention the concentration of SerpinC1 and SerpinG1 are measured and combined to determine whether a patient suffers from stable angina.

The inventors of the present invention have found that particular proteins are differentially expressed between sub-fractions of plasma samples of human patients with ischemic heart disease and reference sub-fractions of plasma samples from human subjects that do not suffer from ischemic heart disease. Reference samples means that the plasma samples are treated in the same manner and sub-fractions LDL and HDL are compared. Preferably, to have a proper comparison between patient and non-patient (yes/no suffering fromstable angina) said human patient and said human subject not suffering from stable angina both experience chest pain, indicative of stable angina. However, the human subject not suffering from ischemic heart disease may have entered the emergency room with chest pain that appeared indicative of stable angina. Of course, the skilled person would understand that other reference samples may also be used in the comparison, for instance those that are obtained from human subjects that are healthy and did not experience chest pain or other symptoms that are related, or that are indicative of stable angina. As outlined above, no rapid and reliable protein-based markers are available in the art that enable a physician or GP to determine quickly whether a human patient suffering from symptoms that are indicative of stable angina, do indeed suffer from stable angina. Such is now presented herein: A plasma sample from a suspected ischemic heart disease patient is fractionated into sub-fractions and the concentration of a single or a set of proteins is determined in said fractions. It is known that the proteins in said fractions are associated with the extracellular vesicles within said fractions. Subsequently, the concentrations are compared to concentrations determined in reference samples from a control human subject and when compared, the determined concentration in said fractions can now tell the physician or GP whether a suspected patient indeed suffers from stable angina.

In a particularly preferred aspect of the invention, said ischemic heart disease is stable angina, and for this the concentrations of SerpinC1 and SerpinG1 are determined. More preferably in the case of stable angina, the concentration of SerpinC1 is determined in the HDL fraction. In another preferred aspect, SerpinC1 in HDL alone appeared indicative.

Also described herein is a kit for performing the method according to the invention, said kit comprising the means for determining the concentration of said protein markers in said fractions. Said means may comprise any suitable means known in the art, such as an immune-bead based process as outlined herein. Further, the kit may comprise instructions and means for the fractionation of a plasma sample to enable the rapid fractionation and subsequent protein concentration determination as outlined herein.

The present invention also relates to a protein marker for the diagnosis of stable angina in a human patient, wherein said protein marker is selected from the group consisting of: human SerpinC1, SerpinG1, CD14 and SerpinF2; or wherein said protein marker is a combination of SerpinC1, SerpinG1, CD14 and SerpinF2. The best performing protein marker in the hands of the inventors appeared to be SerpinC1 that appeared indicative for stable angina in certain plasma sub-fractions (as outlined in detail herein). The AUC value increased when combinations of protein marker concentrations and certain sub-fractions were combined. For stable angina it appeared that the best score was obtained when SerpinC1 was determined in HDL and REX in combination with the concentration of SerpinG1 in TEX (of course compared to their respective values in reference, control samples/fractions).. Hence, the most preferred biomarker for use in most if not all methods of the present invention is human SerpinC1, when its concentration is determined in LDL, and HDL fractions of a plasma sample from a human patient suspected to suffer from stable or unstable angina.

In yet another embodiment, the present invention relates to a method for determining the level of human SerpinC1, SerpinG1, CD14 and/or SerpinF2 in a plasma sample from a human patient experiencing chest pain, said method comprising: obtaining a plasma sample from said patient; fractionating LDL and HDL fractions from said plasma sample; and determining the level of human SerpinC1, SerpinG1, CD14 and/or SerpinF2 in the extracellular vesicles present in said fractions. Preferred protein markers are SerpinC1, CD14 and SerpinG1. Highly preferred is a combination of SerpinC1 and SerpinG1, and more preferably the concentration is determined in HDL.

It should be noted that the above-mentioned embodiments illustrate rather than limit the invention, and that those skilled in the art will be able to design many alternative embodiments.

In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The mere fact that certain measures are recited in mutually different dependent claims does not indicate that a combination of these measures cannot be used to advantage.

### EXAMPLES

### Example 1. Proteomic study on Minerva unstable angina cohort

The MINERVA study (abbreviation for determination of Microvesicle content IN the Emergency Room: diagnostic Value for Acute coronary syndromes) is a single center, prospective cohort study of patients presenting to the emergency department within 24 hours of the onset of chest pain suggestive of acute coronary syndrome (ACS). Between January 2012 and June 2014, over 2000 consecutive patients were enrolled in the Meander Medical Center Amersfoort, the Netherlands, which is a large regional teaching hospital providing healthcare for a population of -300,000 patients. Patients younger than 18 years, patients who were unable or unwilling to give their informed consent and patients with a clear-cut ST-segment elevation MI (STEMI) were not included in this study. All patients were evaluated and managed according to the international guidelines. Along with the routine clinical laboratory measurements directly upon presentation, additional 3x10cc tubes of venous blood were drawn. The plasma component was frozen and stored at -80°C within one hour after sample collection.

### Data acquisition

Detailed patient information was collected and documented in a digital case record form: clinical presentation, medical history, cardiovascular risk factors, current medication use, findings on physical examination, ECG evaluation, blood biochemical parameters and results of additional investigations.

### Diagnosis adjudication

The primary outcome is ACS (i.e. unstable angina, non ST-segment elevation myocardial infarction (NSTEMI) and ST-segment elevation myocardial infarction (STEMI)), adjudicated according to the universal definition of myocardial infarction and the latest ESC guidelines (Hamm et al. ESC Committee for Practice Guidelines. ESC Guidelines for the management of acute coronary syndromes in patients presenting without persistent ST-segment elevation: The Task Force for the management of acute coronary syndromes (ACS) in patients presenting without persistent ST-segment elevation of the European Society of Cardiology (ESC). Eur Heart J 2011 Dec;32(23):2999-3054). The diagnosis of NSTEMI was made when a rise and/or fall in cardiac troponin with at least one value above the 99^{th} percentile was observed in a clinical setting consistent with myocardial ischemia. Unstable angina was diagnosed on the basis of signs and symptoms consistent with myocardial ischemia, accompanied by dynamic ECG changes, evidence of ischemia on functional testing or new coronary angiographic changes. An outcome panel consisting of two cardiologists adjudicated the final diagnosis. In case of uncertainty, a third cardiologist has decided.

### Follow-up

Patients were followed up for at least 1 year after inclusion. Local hospital records were checked for hospitalization (heart failure or unstable angina), myocardial infarction, coronary revascularization and death. Patients were contacted by letter and/or telephone and were asked if they had been admitted to a hospital since inclusion. When this was the case, the hospital records were checked for event verification, also when patients were admitted to a center other than the center of inclusion. Major adverse cardiovascular events were scored, defined as non-fatal MI (STEMI/NSTEMI type 1), coronary revascularization (PCI or CABG), hospital admission for heart failure or unstable angina and/or all cause death during 1 year of follow-up. Verification of the Mass spectrometry discovery was done on 27 definitive UA and 31 controls (see Figure 1).

### Preparation of plasma fractions

Extracellular vesicle plasma sub-fraction isolation using sequential precipitation was generally performed as follows. As previously described (Burnstein et al. Rapid method for the isolation of lipoproteins from human serum by precipitation with polyanions. J Lipid Res 1970;11:583-595), Dextran Sulphate (DS) and Manganese (II) chloride (MnCl₂) solution was used to precipitate LDL and the HDL plasma fractions. Briefly, a stock of DS and MnCl₂ were prepared as 6.5% and 2M solutions respectively. For the Total Extracellular vesicle (TEX) fraction, Xtractt buffer (Cavadis B.V.; 1:4) was added to 125 µl plasma and mixed. The mixture was incubated at 4°C for 30 min and subsequently centrifuged at 4,800g at 4°C for 10 min. This pellet was dissolved in 125 µL Roche lysis buffer (Roche #04719956001) and used in the quantitative magnetic bead assays as the TEX fraction. For precipitation of the LDL fraction, DS stock (1:125) and MnCl₂ stock (1:40) were added into another 125 µL plasma and mixed. The mixed sample was centrifuged for 10 min at 4,800g at 4°C precipitating the LDL fraction pellet. This pellet was dissolved in 125 µL lysis buffer and used in the quantitative magnetic bead assays as the LDL fraction. For the HDL fraction, 60 µL of supernatant above the LDL pellet was transferred to a new tube topped up with 65 µL Phosphate-Buffered-Saline (PBS) and mixed. Next, DS stock (1:10) and MnCl₂ stock (1:10) were added into the 125 µl diluted supernatant and mixed. Subsequently, the sample was incubated for 2 h at 4°C and the sample was centrifuged for 10 min at 4,800g at 4°C to collect the HDL fraction pellet. This pellet was dissolved in 125 µL Roche lysis buffer and used in the quantitative magnetic bead assays as the HDL fraction. For the Remaining Extracellular Vesicles (REX) fraction, 32 µl of Xtractt was added to the remaining supernatant after the HDL to precipitate the remaining extracellular vesicles. After 30 min at 4°C followed by centrifugation at 4000 g for 30 min, the pellet was dissolved in 125 µl Roche lysis buffer with the REX vesicles lysed in this fraction.

### Proteomics

In order to determine the protein content of the vesicle sub-fractions, a mass spectrometry based proteomics run on the LDL fraction of plasma collected from MINERVA patients was performed. Liquid chromatography-tandem mass spectrometry (LC-MS/MS) was used for proteomic analysis as described (Sok Hwee Cheow et al. Simultaneous Enrichment of Plasma Soluble and Extracellular Vesicular Glycoproteins Using Prolonged Ultracentrifugation-Electrostatic Repulsion-hydrophilic Interaction Chromatography (PUC-ERLIC) Approach. MCP 2015 14(6):1657-1671). In short, LC-MS/MS was used to analyze purified proteins from the VLDL-EV fractions. After the VLDL-EV fraction was isolated from the plasma, the fraction was resolved in a lane of a 10% SDS-PAGE gel respectively and subsequently stained with Coomassie blue. The lane was excised and divided into 6 pieces equally for in-gel trypsin digestion; after reduction and alkylation, each piece was digested with Trypsin overnight. Digested peptides were extracted from the gel, dried and then desalted. The peptides were analyzed with a Q-Exactive LC-MS/MS (Thermo Scientific, San Jose, USA). The raw data were converted to mascot generic (mgf) files using Proteome Discoverer ver. 1.4 (Thermo Scientific, San Jose, USA) and then the mgf files were searched against Uniprot human proteome database using an in-house Mascot Server ver. 2.4.1 (Matrix Science, London, UK). Ingenuity Pathway Analysis (IPA^{®}, QIAGEN Redwood City, Build Version 321501M, Content Version 17199142) was used to determine canonical pathways statistically overrepresented in the proteins compared to the Ingenuity database. Proteomics identified 815 individual proteins from which 572 could be mapped to the Ingenuity database.

The top 5 canonical pathways identified were:
- LXR/RXR activation: p= 8.15*10⁻⁵⁶
- Acute Phase response signaling: p= 4.35*10⁻⁴⁵
- FXR/RXR activation: p= 1.58*10⁻⁴²
- Coagulation system: p= 1.23*10⁻³¹
- Complement system: p= 5.12*10⁻²⁷

Proteins selected for verification was done on the following selection criteria: (1) Proteins described as extracellular vesicle protein in Exocarta database (http://exocarta.org/) or identified as extracellular vesicle proteins in earlier studies performed by the inventors; (2) Proteins associated with atherosclerotic disease as the underlying cause of stable coronary artery disease and unstable angina; and (3) Availability of two compatible antibody and recombinant protein that can be used in immuno-bead assay.

As a result, the inventors found the four following proteins to be of particular relevance:
CD14 (*LXR*/*RXR activation pathway) UniProtKB* - *P08571 (CD14_HUMAN*)
SerpinG1 (*Acute Phase response pathway) UniProtKB* - *P05155 (IC1_HUMAN*)
SerpinF2 (*Acute Phase response pathway*/*Coagulation system) UniProtKB* - *P08697 (A2AP_HUMAN*)
SerpinC1 *(Coagulation system) UniProtKB* - *P01008 (ANT3_HUMAN*)

Next to these four, the inventors selected Cystatin C *UniProtKB* - *P01034 (CYTC_HUMAN*), because this protein is not added to any canonical pathway but it is a vesicle protein and is associated with future cardiovascular events. Furthermore, an immuno bead assay is available that is compatible with multiplexing for SerpinG1, CD14 and SerpinF2.

### Example 2. Myomarker stable angina cohort

The MYOMARKER stable coronary artery disease cohort (abbreviation for **MYO**cardial ischemia detection by circulating bio**MARKER**s) is a single center, prospective cohort study of patients, who are evaluated in the Meander Medical Center Cardiology outpatient clinic for (recent onset) suspected symptomatic coronary artery disease, undergoing radionuclide myocardial perfusion imaging (rMPI) as indicated by their own cardiologist. Ahead of rMPI, venous blood (6x10cc) is obtained from the peripheral intravenous cannula, which is inserted as part of standard preparation for rMPI. The plasma component is frozen and stored at -80°C within 1 hour after sample collection. All patients are evaluated and managed according to the international guidelines, by their own cardiologist. Patient enrollment started in August 2014 and continues until at least 1250 patients are participating in the study. At the filing of the present application (Nov 2015) over 800 patients were enrolled. Patients younger than 18 years and patients who are unable or unwilling to give their informed consent are not included in this study.

### Data acquisition

Patient information is collected and documented in a digital case record form: clinical presentation, medical history, cardiovascular risk factors, current medication use, ECG evaluation, blood biochemical parameters and results of additional investigations.

### Endpoint adjudication

Objectified ischemic coronary artery disease, as determined by radionuclide myocardial perfusion imaging (Rubidium-82) and adjudicated by a panel of at least two nuclear medicine physicians.

### Follow-up

Patients are followed up for at least 1 year after inclusion. Local hospital records were checked for hospitalization (heart failure or unstable angina), additional cardiac and/or coronary imaging, coronary revascularization, myocardial infarction and death. Patients were contacted by letter and/or telephone and were asked if they had been admitted to a hospital since inclusion. When this was the case, the hospital records were checked for event verification, also when patients were admitted to a center other than the center of inclusion. Major adverse cardiovascular events (MACE) were scored, defined as non-fatal MI (STEMI/NSTEMI type 1), coronary revascularization (PCI or CABG), hospital admission for heart failure or unstable angina and/or all cause death during 1 year of follow-up.

### Preparation of plasma fractions and proteomics

Preparation of plasma fractions and proteomics was performed in the same way as in example 1.

### Example 3. Localization of proteins associated with extracellular vesicles

In order to determine if the five proteins selected were present in or on extracellular vesicles (EV), density gradient ultracentrifugation was performed. VLDL-EV, HDL-EV, REX-EV and TEX-EV fractions were isolated from 8 mL plasma as mentioned under example 1. Each of the precipitates (LDL, HDL, REX, TEX) was re-suspended in 500 µl PBS and used for the density gradient centrifugation. In order to make the density gradient buffer, 5 solutions containing different concentrations of OptiPrep^{™} medium (OptiPrep, Axis-shiled #1114542) were prepared with 10x PBS pH 7.4 (Ambion^{®}, Life Technologies #AM9265) and ddH₂O. The working solutions comprised 5%, 10%, 20%, 30% and 40% OptiPrep and 1xPBS respectively. The solutions were added and overlaid into the ultracentrifuge tubes (Beckman Coulter #344059) sequentially from the highest density one (with the highest concentration of OptiPrep) to the lowest density one (with the lowest concentration of OptiPrep). Subsequently, the pellets isolated of each fraction was carefully added in the ultracentrifuge tubes on top of the density gradient buffers. Then they were spun at 200,000g for 18h at 4°C with low acceleration and low brake (Beckman Coulter #Optima XL-90 Ultracentrifuge #SW 41 Ti Rotor). Afterwards, 10 different gradient sub-fractions (1 mL per sub-fraction) were harvested sequentially (from top to bottom) in each ultracentrifuge tube and transferred into 1.5 mL Eppendorf tubes. After thorough vortexing of the sub-fractions, 900 µL of each sub-fraction was transferred into a new ultracentrifuge tube together with 7 mL 0.1% BSA buffer (Sigma Aldrich # 05470, in 1x PBS, m/v), and then centrifuged at 200,000g for 1h at 4°C. The pellets were re-suspended in 200 µL Roche lysis buffer for protein level measurement.

Quantitative measurement using a beads-based immunoassay, was done as described elsewhere (Kanhai et al. Microvesicle protein levels are associated with increased risk for future vascular events and mortality in patients with clinically manifest vascular disease. Int J Cardiol 2013 168:2358-2363). In short, the beads (Luminex #MagPlex-C Microspheres, MC100) were conjugated with the selected antibodies to synthesize the bead-capture antibody complex. Samples were incubated with the bead-capture antibody complex and subsequently with the biotinylated antibodies to detect the captured protein. Streptavidin-phycoerythrin (SA-PE, BD bioscience#554061) was added to quantify the concentration of captured protein. Standard curves were correlated to the SA-PE signal and dilution of homologous recombinant proteins. The Bio-Plex^{®} 200 Systems (Bio-Rad#171-000201) were used for measurement and data process. The antibodies and recombinant proteins used in detection were listed as follow. For SerpinC1: Antithrombin III antibody (NOVUS #NBP1-05149), human SerpinC1 Biotinylated affinity purified PAb (R&D #BAF1267), recombinant human SerpinC1 (R&D System #1267-PI-010); for SerpinF2: anti-human SerpinF2 (R&D Systems #MAB1470), biotinylated anti-human SerpinF2 (R&D Systems #BAF1470), recombinant human SerpinF2 (R&D Systems #1470-PI-010). For Cystatin C were anti-Human Cystatin C (R&D Systems # MAB11962), biotinylated anti-Human Cystatin C (R&D Systems # BAM11961) and recombinant Human Cystatin C (R&D Systems #1196-PI). For CD14, anti-Human CD14 (R&D Systems #MAB3832), biotinylated anti-Human CD14 (R&D Systems #BAF383) and recombinant Human CD14 (R&D Systems #383-CD) were used and for SerpinG1, anti-Human SerpinG1 (R&D Systems #MAB2488), biotinylated anti-Human SerpinG1 (R&D Systems #BAF2488) and recombinant Human SerpinG1 (R&D Systems #2488-PI) were used. This quantitative measurement of protein levels is also referred to as the Bioplex assay.

After density gradient ultracentrifugation each fraction was used in the Bioplex assay to measure the protein concentration in each fraction as well as the density in each of the fractions. For each of the 4 plasma sub-fractions and the measurements of CD14, SerpinG1, SerpinF2, Cystatin C, SerpinC1 in each of the 10 density gradient fractions is shown in Figure 2. This table shows that the proteins are indeed associated with extracellular vesicles as only vesicles can float in such density gradients. LDL particles also float but due to the high lipid content their density is lower than 1. HDL have a density of 1-1.2. The REX and TEX fraction do not contain lipoparticles. In the table of Figure 2 it is shown that LDL particles do not contain the EV proteins as LDL particles float all below a density < 1. For HDL, this is less clear as density of EV partly coincides with HDL although the EV proteins are also present in the higher density region where no HDL particles are present. For the REX and TEX fraction, the proteins all float with extracellular vesicles with a density between 1 - 1.2 (TEX) and between 1 - 1.8.

### Example 4. Validation of extracellular vesicle proteins for diagnosis of stable coronary artery disease

### Statistical Analyses for Stable Angina (Myomarker cohort)

Differences in baseline-characteristics were analyzed using Chi-square test for categorical variables, T-tests for normally distributed continuous variables and Mann-Whitney-U-tests for continuous variables that were not normally distributed. To calculate the odds ratio and enable the direct comparison between different proteins and fractions, EV-protein levels were converted into standard deviation units, or the z-score, by using the observed value minus the mean value, divided by the standard deviation. Selection of the best combination of markers and sub-fractions was done using Logistic regression analysis with forward selection based upon the Chi-square test and based upon Akaike Information Criterion (AIC). Receiver-operating characteristic (ROC) analysis was performed for AUC and optimal calculated cutoff for NPV and PPV. All statistics was achieved by SPSS^{®} (IBM^{®}, Version 22) and Rstudio using R software for statistical computing version 3.1.2.

### Diagnosis of stable angina

Although unstable angina is thought to be much more coagulation-associated then stable angina, the underlying disease process is the same: atherosclerosis.

For this, the 5 extracellular vesicle proteins SerpinF2, SerpinG1, SerpinC1, CD14, and Cystatin C were measured in the plasma fractions LDL, HDL, REX and TEX in a cohort of 30 stable coronary artery disease patients of the Myomarker cohort and 30 sex-, age, history-, and medication-matched controls of the same cohort using sequential precipitation as described under example 1 above and an immuno-bead assay as described under example 3 above. The best performing individual marker/fraction pairs and marker/ratio pairs for identification of stable angina are listed in table 1. Table 1 lists the most significant and best performing marker/fraction pairs and marker/ratio pairs, which on their own, and with no need for combination with further marker/fraction pairs or marker/ratio pairs, provide a statistically relevant identification of subjects suffering from stable angina. Table 1 shows the AUC values upper and lower values obtained in the ROC plots of these most significant and best performing individual marker/fraction pairs or marker/ratio pairs. The ROC plots are performed on the data from the above-mentioned patient and control groups of the Myomarker cohort. All listed combinations have a p-value of 0.05 or less. The abbreviations and the nomenclature of table 1are: c1=SerpinC1, g1=SerpinG1, f2=SerpinF2, cc=Cystatin C, cd14=CD14, Idl=Low-Density lipoprotein, hdl=High-Density Lipoprotein, rex=remaining fraction, tex=total fraction. By way of example "c1 hdl/rex" means that the ROC plot is based on the ratio of a value derived from the concentration of SerpinC1 in plasma fraction HDL over plasma fraction REX, where the ROC plot is performed on the data from the above-mentioned patients and control groups of the Myomarker cohort. All listed combinations have a p-value of 0.05 or less.

**Table 1:**

| **Markers** | **AUC** | **Lower** | **upper** |
|---|---|---|---|
| **c1-hdl/tex** | 0.842 | 0.741 | 0.944 |
| **c1-hdl/rex** | 0.828 | 0.718 | 0.938 |
| **c1-hdl** | 0.812 | 0.7 | 0.924 |
| **f2-ldl/hdl** | 0.811 | 0.695 | 0.927 |
| **cd14-hdl/tex** | 0.806 | 0.692 | 0.919 |
| **cd14-ldl/hdl** | 0.764 | 0.638 | 0.891 |
| **cd14-hdl/rex** | 0.733 | 0.605 | 0.862 |
| **c1-rex/tex** | 0.727 | 0.598 | 0.855 |
| **c1-ldl/hdl** | 0.719 | 0.589 | 0.849 |
| **f2-hdl/tex** | 0.717 | 0.584 | 0.851 |
| **cd14-hdl** | 0.716 | 0.584 | 0.847 |
| **c1-tex** | 0.702 | 0.567 | 0.837 |
| **f2-ldl/rex** | 0.689 | 0.546 | 0.832 |
| **cc-hdl/tex** | 0.683 | 0.547 | 0.819 |
| **g1-rex/tex** | 0.662 | 0.52 | 0.804 |
| **cc-hdl/rex** | 0.654 | 0.509 | 0.8 |
| **cc-ldl/hdl** | 0.653 | 0.513 | 0.794 |

The best performing combinations of two marker/fraction pairs and marker/ratio pairs for identification of stable angina are listed in table 2. Combination of two marker/fractions and/or marker/ratios means that two different values derived from the concentration of at least one protein marker is determined. The two different values can for example be values derived from the concentration of the same marker but in different fractions and/or ratios or it can be of different markers in the same or different fractions and/or ratios. Table 2 lists the most significant and best performing combinations of two marker/fraction pairs and marker/ratio pairs and show the AUC values as obtained in the ROC plots of these most significant and best performing combinations of marker/fraction pairs and/or marker/ratio pairs. The ROC plots were performed on the data from the above-mentioned patient and control groups of the Myomarker cohort. All listed combinations have a p-value of 0.05 or less. The abbreviations and the nomenclature of table 2 is the same as in table 1. However, in table 2 the "j" sign is missing between the fractions of which a ratio is taken. By way of example, "c1ldlhdl" means the ratio of a value derived from the concentration of SerpinC1 in fraction LDL over HDL..

**Table 2:**

| Markers | AUC | Markers | AUC | Markers | AUC |
|---|---|---|---|---|---|
| clhdl+f2ldlhdl | 0.881 | cd14ldlhdl+ccrextex | 0.784 | cltex+f2rextex | 0.703 |
| c1hdltex+cchdltex | 0.876 | cd14hdlrex+c1ldlhdl | 0.783 | cltex+ccrextex | 0.703 |
| cd141dltex+clhdltex | 0.874 | cd14ldlhdl+ccldlrex | 0.783 | c1ldltex+f2hdltex | 0.703 |
| cd14rextex+c1hdltex | 0.873 | cd14ldlhdl+g1hdl | 0.781 | cd14hdltex+c1ldl | 0.703 |
| c1hdltex+ccldltex | 0.873 | cd14ldlhdl+ccldlhdl | 0.78 | f2rextex+cchdlrex | 0.703 |
| c1hdltex+c1rextex | 0.873 | cd14hdlrex+f2ldlrex | 0.78 | f2ldlrex+g1hdlrex | 0.703 |
| c1hdlrex+f2ldlhdl | 0.872 | cd14hdl+cchdlrex | 0.779 | f2ldlrex+g1hdl | 0.702 |
| f2ldlhdl+g1hdl | 0.872 | cd14ldlhdl+cd14hdlrex | 0.779 | cd14hdltex+ccldlhdl | 0.701 |
| c1hdltex+g1rextex | 0.869 | cd14ldlhdl+cctex | 0.779 | c1tex+g1ldltex | 0.701 |
| f2ldlhdl+g1ldl | 0.868 | cd 14h d Itex+cd 14rextex | 0.779 | c1tex+g1hdltex | 0.701 |
| c1tex+c1hdlrex | 0.867 | c1tex+c1ldlhdl | 0.778 | cltex+cchdl | 0.701 |
| f2ldl+f2hdl | 0.861 | cd141dlhdl+cd14ldlrex | 0.777 | cltex+ccldlrex | 0.701 |
| c1hdlrex+c1hdltex | 0.86 | cd14ldlhdl+c1ldlrex | 0.776 | cltex+cchdltex | 0.701 |
| c1hdltex+ccrextex | 0.859 | cd14ldlhdl+c1ldltex | 0.776 | c1ldlhdl+g1ldlrex | 0.701 |
| c1hdl+c1tex | 0.859 | cd14ldlhdl+f2ldltex | 0.776 | clrextex+ccldl | 0.701 |
| c1hdltex+g1hdltex | 0.857 | cd14hdl+cd14rex | 0.774 | glrex+cchdlrex | 0.7 |
| f2rex+f2ldlhdl | 0.857 | cd14hdltex+ccrex | 0.774 | cd14hdltex+f2hdl | 0.699 |
| c1hdlrex+g1rextex | 0.857 | cd14hdltex+f2hdltex | 0.774 | c1rextex+g1ldl | 0.699 |
| c1hdlrex+c1rextex | 0.856 | cd14ldl+cd14hdl | 0.773 | cltex+f2hdlrex | 0.699 |
| c1ldlhdl+f2ldlhdl | 0.856 | cd14hdl+cd14ldlhdl | 0.773 | cd14tex+cd14hdltex | 0.699 |
| f2hdl+f2ldlhdl | 0.854 | cd14hdl+c1tex | 0.772 | cd14hdltex+c1ldltex | 0.699 |
| c1hdltex+f2ldltex | 0.853 | cd14rex+cd14hdlrex | 0.772 | cltex+f2hdltex | 0.699 |
| f2ldlhdl+g1ldlrex | 0.853 | cd14ldlrex+cd14hdlrex | 0.772 | cltex+f2tex | 0.698 |
| c1hdlrex+g1hdl | 0.852 | cd14ldl+cd14ldlhdl | 0.772 | cltex+ccldl | 0.698 |
| cd14hdltex+c1hdlrex | 0.852 | cd14hdlrex+g1rex | 0.771 | cd14hdltex+cchdlrex | 0.698 |
| c1hdlrex+f2rextex | 0.852 | cd14hdltex+g1hdlrex | 0.771 | f2ldlrex+cchdl | 0.698 |
| f2ldlhdl+g1hdlrex | 0.852 | cd14ldlhdl+g1ldl | 0.771 | f2ldlrex+g1tex | 0.698 |
| cd14hdlrex+c1hdl | 0.851 | f2ldlrex+cchdlrex | 0.771 | cd14tex+cchdl | 0.697 |
| f2ldl+f2ldlhdl | 0.851 | cd14hdl+cd14hdlrex | 0.77 | glrextex+cchdltex | 0.697 |
| c1rex+c1hdlrex | 0.85 | c1hdltex+f2ldlrex | 0.77 | cd14ldlrex+f2ldlrex | 0.697 |
| c1hdlrex+g1hdltex | 0.85 | cd14ldlhdl+g1hdlrex | 0.769 | f2hdl+f2ldlrex | 0.697 |
| c1hdlrex+g1ldltex | 0.85 | cd14ldlhdl+ccldl | 0.769 | cd14hdltex+c1rextex | 0.696 |
| cd14ldlhdl+c1hdlrex | 0.85 | cd14ldlhdl+f2rex | 0.769 | ccldlrex+cchdlrex | 0.696 |
| cd14hdltex+c1hdltex | 0.849 | c1ldlhdl+g1tex | 0.768 | c1ldltex+f2ldlrex | 0.696 |
| c1ldlrex+c1hdlrex | 0.849 | cd14ldlhdl+c1ldl | 0.768 | f2ldl+f2hdltex | 0.696 |
| c1hdlrex+ccldltex | 0.849 | cd14ldlhdl+g1rex | 0.768 | cchdl+cchdlrex | 0.694 |
| c1hdlrex+cchdltex | 0.849 | cd14ldlhdl+f2hdl | 0.768 | c1rextex+cchdlrex | 0.694 |
| cd14ldltex+cd14hdltex | 0.848 | c1ldlhdl+cchdlrex | 0.767 | cd14hdltex+f2ldlrex | 0.694 |
| f2ldlhdl+g1rex | 0.848 | cd14ldlhdl+g1ldlhdl | 0.766 | cd14hdltex+f2ldltex | 0.694 |
| c1hdl+c1hdltex | 0.847 | c1ldlhdl+f2ldlrex | 0.763 | c1ldl+f2ldlrex | 0.693 |
| c1hdltex+f2hdl | 0.847 | cd14rex+cd14ldlhdl | 0.762 | c1rextex+f2ldlrex | 0.693 |
| cd14rextex+c1hdlrex | 0.847 | cd14ldlhdl+f2hdlrex | 0.762 | f2ldlrex+g1hdltex | 0.693 |
| c1rex+f2ldlhdl | 0.847 | cd14ldlhdl+cchdl | 0.761 | cd14hdltex+cchdl | 0.692 |
| c1hdlrex+g1tex | 0.847 | cd14ldlhdl+g1ldlrex | 0.761 | c1rextex+f2hdl | 0.692 |
| c1hdltex+g1ldltex | 0.846 | cd14ldlhdl+ccrex | 0.76 | c1rextex+ccldlhdl | 0.692 |
| c1hdltex+f2ldlhdl | 0.845 | cd14hdltex+g1ldlrex | 0.76 | cchdltex+ccrextex | 0.692 |
| c1ldlhdl+c1hdlrex | 0.844 | cd14hdl+cctex | 0.759 | f2rex+f2ldlrex | 0.692 |
| f2ldltex+f2hdltex | 0.844 | cd14ldlhdl+c1rex | 0.758 | f2ldlrex+g1ldltex | 0.692 |
| cd14hdl+f2ldlhdl | 0.844 | cd14ldlhdl+f2tex | 0.757 | f2rextex+cchdl | 0.691 |
| cd14ldlhdl+c1hdltex | 0.844 | cd14ldlhdl+f2hdltex | 0.757 | c1rextex+cctex | 0.691 |
| c1hdlrex+ccrex | 0.844 | cd14hdlrex+cctex | 0.756 | cd 14tex+clrextex | 0.69 |
| c1hdlrex+ccrextex | 0.844 | cd14hdlrex+g1hdl | 0.752 | glrextex+ccldlhdl | 0.69 |
| c1ldl+c1hdltex | 0.843 | cd14hdtex+g1ldl | 0.752 | f2ldl+f2ldlrex | 0.69 |
| cd14hdl+c1hdlrex | 0.843 | cd14tex+c1ldlhdl | 0.751 | g1rextex+cchdlrex | 0.69 |
| c1hdltex+g1hdlrex | 0.843 | cd14hdlrex+g1ldlrex | 0.751 | cd14hdltex+cchdltex | 0.69 |
| cd14hdlrex+c1hdlrex | 0.843 | cd14hdlrex+f2hdlrex | 0.749 | cd14rex+cchdlrex | 0.689 |
| c1hdlrex+cctex | 0.843 | cd14hdl+c1rextex | 0.748 | f2ldlrex+ccldlhdl | 0.689 |
| cd14ldltex+c1hdlrex | 0.843 | cd14hdrex+g1rextex | 0.748 | ccldlhdl+ccldlrex | 0.689 |
| cd14ldlrex+c1hdlrex | 0.843 | cd14hdlrex+g1ldl | 0.747 | cd14rex+f2ldlrex | 0.689 |
| c1hdltex+f2tex | 0.842 | cd14hdlrex+g1tex | 0.747 | f2rextex+ccldlhdl | 0.688 |
| c1hdltex+f2hdltex | 0.841 | cd 14hdl rex+c1rextex | 0.746 | g1lhdl+cchdl | 0.688 |
| c1hdltex+f2rextex | 0.841 | cd14tex+cd14hdlrex | 0.746 | cd14rextex+c1rextex | 0.688 |
| c1tex+c1hdltex | 0.841 | cd14hdlrex+cchdl | 0.746 | f2ldlrex+f2ldltex | 0.688 |
| c1hdlrex+g1rex | 0.841 | c1tex+g1hdl | 0.746 | f2ldlrex+ccrextex | 0.688 |
| c1hdltex+f2rex | 0.84 | cd14hdltex+c1rex | 0.744 | f2ldlrex+g1ldl | 0.688 |
| c1hdl+c1hdlrex | 0.84 | cd14hdltex+ccldl | 0.744 | f2ldlrex+g1rex | 0.688 |
| cd14hdlrex+f2ldlhdl | 0.839 | cd14hdlrex+ccldlhdl | 0.744 | f2ldlrex+g1ldlrex | 0.688 |
| c1ldlhdl+c1ldlrex | 0.839 | cd14hdlrex+f2hdl | 0.744 | cd14rextex+f2ldlrex | 0.687 |
| c1hdltex+ccldlrex | 0.838 | cd14hdl+cd14hdltex | 0.743 | f2ldlrex+ccldl | 0.687 |
| c1hdlrex+ccldlhdl | 0.838 | cd 14hd Irex+ccrex | 0.743 | g1ldlhdl+cchdltex | 0.686 |
| c1hdlrex+f2tex | 0.838 | cd14hdlrex+g1hdlrex | 0.743 | ccldlhdl+cchdltex | 0.686 |
| c1hdlrex+g1hdlrex | 0.838 | cd14ldlhdl+f2ldlrex | 0.743 | f2ldlrex+cchdltex | 0.685 |
| c1ldltex+c1hdltex | 0.837 | cd14ldl+c1ldlhdl | 0.742 | cd14ldl+f2ldlrex | 0.685 |
| cd14rex+c1hdlrex | 0.837 | cd14hdl+f2rextex | 0.741 | c1rex+f2ldlrex | 0.685 |
| c1hdltex+ccldl | 0.836 | c1ldlhdl+c1ldltex | 0.741 | f2ldlrex+g1ldlhdl | 0.685 |
| c1hdl+g1tex | 0.836 | cd14hdl+g1ldltex | 0.741 | f2ldlrex+ccrex | 0.685 |
| c1hdlrex+g1ldlhdl | 0.836 | cd14hdl+cchdltex | 0.741 | f2ldlrex+g1rextex | 0.685 |
| c1hdlrex+g1ldl | 0.836 | cd14hdlrex+f2rextex | 0.741 | g1hdltex+cchdltex | 0.684 |
| cd14ldl+c1hdltex | 0.834 | cd14hdlrex+cd14hdltex | 0.741 | g1tex+cchdlrex | 0.684 |
| c1hdltex+ccrex | 0.834 | cd14hdlrex+ccrextex | 0.741 | f2ldlrex+f2rextex | 0.684 |
| c1ldl+c1hdlrex | 0.834 | c1tex+g1rex | 0.741 | cd14ldltex+f2ldlrex | 0.684 |
| c1hdltex+g1ldlrex | 0.834 | cd14hdl+g1tex | 0.74 | f2ldlrex+ccldltex | 0.684 |
| c1hdlrex+f2hdl | 0.834 | cd14hdlrex+f2rex | 0.74 | g1ldltex+cchdltex | 0.683 |
| c1hdlrex+g1ldlrex | 0.834 | cd14hdlrex+g1ldltex | 0.74 | cd14ldltex+cchdltex | 0.683 |
| c1hdlrex+ccldlrex | 0.834 | cd14hdlrex+f2tex | 0.74 | cd14tex+f2ldlrex | 0.683 |
| cd14tex+c1hdlrex | 0.834 | cd14hdlrex+ccldltex | 0.74 | f2tex+f2ldlrex | 0.683 |
| f2ldlhdl+cchdlrex | 0.833 | cd14ldl+cd14hdlrex | 0.739 | ccldlrex+cchdltex | 0.681 |
| c1hdlrex+f2hdlrex | 0.832 | cd14hdlrex+c1rex | 0.739 | c1ldlrex+cchdlrex | 0.681 |
| c1ldltex+c1hdlrex | 0.832 | cd14hdlrex+cd14rextex | 0.739 | c1rextex+f2rex | 0.681 |
| c1hdlrex+cchdl | 0.832 | cd14hdl+g1hdltex | 0.738 | c1rextex+g1tex | 0.681 |
| cd14hdltex+c1hdl | 0.831 | cd14hdl+g1ldlhdl | 0.738 | f2hdltex+g1rextex | 0.681 |
| c1hdlrex+f2rex | 0.831 | c1tex+c1ldltex | 0.738 | cd14ldl+cchdltex | 0.68 |
| c1hdlrex+ccldl | 0.831 | cd14hdl+g1rextex | 0.737 | c1rextex+g1ldlrex | 0.68 |
| c1hdlrex+cchdlrex | 0.831 | cd14hdlrex+c1ldlrex | 0.737 | g1ldltex+ccldlhdl | 0.679 |
| cd14ldl+f2ldlhdl | 0.831 | cd14hdlrex+ccldlrex | 0.737 | cd14ldlrex+cd14hdltex | 0.679 |
| c1hdltex+g1hdl | 0.83 | cd14ldltex+cd14hdlrex | 0.737 | gltex+ccldlhdl | 0.678 |
| c1rex+c1hdltex | 0.83 | cd14hdlrex+g1hdltex | 0.737 | c1rextex+g1rex | 0.678 |
| cd14ldl+c1hdlrex | 0.83 | c1ldlhdl+f2ldl | 0.737 | glldlrex+cchdlrex | 0.678 |
| f2ldlhdl+g1rextex | 0.83 | cd14hdltex+c1ldlhdl | 0.736 | c1rextex+cchdl | 0.677 |
| c1hdl+f2rextex | 0.829 | f2hdl+f2tex | 0.736 | cd14rex+c1rextex | 0.677 |
| clhdl+clrextex | 0.829 | cd14hdlrex+g1ldlhdl | 0.736 | f2tex+cchdlrex | 0.677 |
| c1hdl+cchdltex | 0.829 | cd14hdlrex+cchdltex | 0.736 | cchdlrex+ccrextex | 0.677 |
| c1hdl+g1ldltex | 0.829 | c1ldlhdl+c1rextex | 0.734 | f2ldlrex+cctex | 0.677 |
| c1hdlrex+f2ldltex | 0.829 | c1ldlhdl+cchdltex | 0.734 | cchdlrex+cchdltex | 0.677 |
| cd14ldlhdl+c1hdl | 0.828 | cd14hdl+cd14rextex | 0.734 | f2ldltex+f2rextex | 0.677 |
| c1hdl+c1rex | 0.828 | cd14hdlrex+ccldl | 0.733 | glhdltex+ccldlhdl | 0.676 |
| c1hdltex+g1ldlhdl | 0.827 | cltex+cchdlrex | 0.733 | c1rextex+f2hdlrex | 0.675 |
| c1hdl+g1hdltex | 0.827 | cd14hdl+ccldl | 0.732 | cd14hdltex+f2rextex | 0.674 |
| cltex+f2ldlhdl | 0.827 | cd14rex+c1ldlhdl | 0.732 | cd14tex+ccldlhdl | 0.674 |
| c1ldlrex+c1hdltex | 0.826 | c1ldlhdl+cctex | 0.732 | ccldlhdl+ccldltex | 0.674 |
| cd14rex+c1hdltex | 0.826 | cd14hdl+cd14ldltex | 0.732 | cchdl+ccrex | 0.673 |
| cd14rextex+c1hdl | 0.826 | cd14hdl+ccldlrex | 0.732 | c1ldl+cchdlrex | 0.673 |
| c1hdl+g1ldlhdl | 0.826 | c1ldlhdl+f2hdlrex | 0.732 | ccldlhdl+cchdlrex | 0.673 |
| cd14ldl+c1hdl | 0.826 | cd14hdltex+f2rex | 0.732 | c1rextex+f2rextex | 0.672 |
| cd14tex+c1hdl | 0.826 | cd14hdl+g1hdl | 0.731 | cd14rextex+ccldlhdl | 0.672 |
| cd14ldlhdl+f2ldlhdl | 0.825 | c1ldlhdl+f2tex | 0.73 | cd14ldltex+ccldlhdl | 0.672 |
| c1hdltex+g1ldl | 0.824 | c1rextex+g1rextex | 0.73 | cd14ldlrex+cchdlrex | 0.672 |
| c1hdltex+g1rex | 0.824 | c1rextex+ccrextex | 0.73 | cd14hdltex+f2ldl | 0.672 |
| c1hdl+g1rextex | 0.824 | cd14hdlrex+cchdlrex | 0.73 | f2ldl+cchdlrex | 0.672 |
| f2ldlhdl+ccldlrex | 0.824 | cd14hdl+f2ldlrex | 0.73 | f2hdltex+g1hdltex | 0.671 |
| cdl4ldlrex+f2ldlhdl | 0.824 | cd14ldlhdl+f2ldl | 0.73 | ccldl+cchdl | 0.671 |
| f2tex+f2ldlhdl | 0.824 | cd14hdl+ccldltex | 0.729 | cctex+ccldlhdl | 0.671 |
| c1hdltex+f2hdlrex | 0.823 | cd14hdl+c1ldlhdl | 0.729 | cd14hdltex+f2tex | 0.671 |
| cd14hdlrex+c1hdltex | 0.823 | cd14hdl+ccrex | 0.729 | cd14tex+cchdlrex | 0.671 |
| c1hdl+ccldltex | 0.823 | cd14hdlrex+c1ldltex | 0.729 | cd14hdltex+c1ldlrex | 0.67 |
| c1hdl+ccrextex | 0.823 | cd14hdl+g1hdlrex | 0.729 | cd 14hd Itex+gltex | 0.67 |
| cd14rex+c1hdl | 0.823 | ccldltex+cchdltex | 0.729 | cd14ldlrex+c1rextex | 0.67 |
| cd14ldltex+c1hdl | 0.823 | c1ldlhdl+ccrex | 0.728 | cd14rex+ccldlhdl | 0.669 |
| c1hdl+c1ldltex | 0.823 | c1rex+c1ldlhdl | 0.728 | ccldlhdl+ccrextex | 0.669 |
| cd14hdltex+f2ldlhdl | 0.823 | cd14hdl+ccldlhdl | 0.728 | g1ldl+cchdltex | 0.668 |
| f2ldlhdl+f2hdltex | 0.823 | c1rextex+ccldltex | 0.728 | g1ldlrex+cchdltex | 0.668 |
| c1hdlrex+f2ldlrex | 0.823 | c1ldlhdl+g1hdl | 0.727 | c1tex+f2ldl | 0.668 |
| c1ldlrex+f2ldlhdl | 0.823 | c1tex+g1ldl | 0.727 | f2ldltex+cchdlrex | 0.668 |
| f2ldlhdl+gltex | 0.823 | c1ldlhdl+g1ldlhdl | 0.726 | cd14rextex+cchdlrex | 0.667 |
| f2ldlhdl+cctex | 0.823 | cd14hdlrex+c1ldl | 0.726 | f2ldltex+cchdl | 0.667 |
| cd14ldlhdl+c1ldlhdl | 0.822 | cd14hdlrex+f2hdltex | 0.725 | f2tex+g1hdl | 0.666 |
| c1hdlrex+f2hdltex | 0.822 | cd14ldltex+c1ldlhdl | 0.724 | f2hdl+f2rextex | 0.666 |
| c1hdltex+g1tex | 0.821 | cd14hdl+ccrextex | 0.724 | glldl+cchdlrex | 0.666 |
| f2ldlhdl+f2ldltex | 0.821 | c1ldlhdl+f2rextex | 0.723 | cltex+f2ldltex | 0.665 |
| f2ldlhdl+g1ldltex | 0.821 | cltex+f2hdl | 0.723 | ccldl+ccldlhdl | 0.664 |
| c1hdltex+ccldlhdl | 0.821 | c1ldlhdl+g1hdlrex | 0.722 | f2hdl+ccldlhdl | 0.664 |
| cd14ldlrex+c1hdltex | 0.821 | c1ldlhdl+g1rextex | 0.722 | cd14ldltex+f2rextex | 0.663 |
| c1hdlrex+f2ldl | 0.821 | cd14hdl+c1ldl | 0.722 | cd14rextex+f2rextex | 0.663 |
| cd14hdl+cd14tex | 0.82 | cd14rextex+c1ldlhdl | 0.721 | f2rextex+ccrextex | 0.663 |
| cd14ldlrex+c1hdl | 0.82 | cd14hdl+f2tex | 0.72 | c1ldl+c1rextex | 0.663 |
| c1rextex+f2ldlhdl | 0.82 | cd14hdl+c1ldltex | 0.72 | g1ldlrex+ccldlhdl | 0.663 |
| f2ldlhdl+cchdl | 0.82 | c1ldlhdl+g1hdltex | 0.72 | g1hdlrex+cchdlrex | 0.663 |
| f2ldlhdl+f2rextex | 0.82 | c1ldlhdl+ccldltex | 0.72 | cd14rextex+ccrex | 0.662 |
| cd14hdltex+ccrextex | 0.819 | c1ldlhdl+ccrextex | 0.72 | c1rex+cchdltex | 0.662 |
| f2ldlhdl+g1hdltex | 0.819 | c1ldlhdl+g1rex | 0.72 | c1rex+cchdlrex | 0.662 |
| cdl4tex+f2ldlhdl | 0.817 | c1ldlhdl+ccldlhdl | 0.72 | g1ldltex+cchdl | 0.661 |
| c1hdl+cctex | 0.817 | cd14hdl+f2hdlrex | 0.72 | g1rextex+cchdl | 0.661 |
| f2ldlrex+f2hdlrex | 0.817 | c1tex+g1hdlrex | 0.72 | g1ldlhdl+cchdlrex | 0.661 |
| c1hdl+cchdlrex | 0.817 | c1ldlhdl+g1ldltex | 0.719 | f2rextex+ccldltex | 0.66 |
| c1hdl+g1ldl | 0.816 | c1rextex+g1ldlhdl | 0.719 | cchdl+cchdltex | 0.66 |
| c1hdl+g1hdlrex | 0.816 | cd14ldl+c1rextex | 0.719 | f2rex+ccldlhdl | 0.66 |
| c1hdl+cchdl | 0.816 | cd14hdl+f2hdltex | 0.718 | cd14ldl+ccldlhdl | 0.66 |
| f2ldlhdl+ccldltex | 0.816 | cd14hdl+cchdl | 0.718 | ccrex+cchdltex | 0.66 |
| f2ldlhdl+ccldlhdl | 0.816 | cd14hdltex+f2hdlrex | 0.718 | c1ldl+ccldlhdl | 0.659 |
| f2ldlhdl+ccrex | 0.815 | cd14hdl+f2ldltex | 0.718 | g1ldl+ccldlhdl | 0.659 |
| c1hdl+g1rex | 0.815 | cd14hdlrex+f2ldl | 0.718 | g1rex+gltex | 0.659 |
| f2ldlhdl+g1ldlhdl | 0.815 | c1ldlhdl+f2hdltex | 0.717 | g1hdltex+cchdl | 0.657 |
| c1hdl+ccrex | 0.814 | c1rextex+g1hdltex | 0.717 | g1tex+cchdl | 0.657 |
| c1hdl+g1hdl | 0.814 | cchdl+cctex | 0.717 | c1rextex+f2tex | 0.657 |
| c1hdl+g1ldlrex | 0.813 | cd14hdl+g1ldlrex | 0.717 | g1hdlrex+cchdl | 0.657 |
| c1hdl+ccldl | 0.813 | c1tex+g1rextex | 0.717 | f2hdltex+g1ldltex | 0.656 |
| f2ldlhdl+cchdltex | 0.813 | f2ldlrex+f2hdltex | 0.717 | f2hdltex+cchdl | 0.656 |
| c1hdl+f2hdlrex | 0.813 | c1ldlhdl+f2rex | 0.716 | f2ldltex+ccldlhdl | 0.656 |
| f2ldlhdl+ccldl | 0.813 | c1ldlhdl+cchdl | 0.716 | f2ldltex+f2hdlrex | 0.655 |
| c1hdl+f2ldltex | 0.813 | c1rextex+g1ldltex | 0.716 | c1rextex+f2ldl | 0.655 |
| f2ldlhdl+f2ldlrex | 0.813 | c1tex+c1ldlrex | 0.716 | glrex+ccldlhdl | 0.654 |
| f2ldlhdl+f2hdlrex | 0.813 | f2ldlrex+ccldlrex | 0.716 | glrex+cchdltex | 0.654 |
| cd14tex+c1hdltex | 0.812 | cd14hdlrex+f2ldltex | 0.716 | cd14ldl+cchdlrex | 0.654 |
| c1hdl+f2rex | 0.812 | cd14hdltex+g1rex | 0.715 | f2hdl+cchdlrex | 0.654 |
| c1hdltex+cctex | 0.812 | cd14hdl+cd14ldlrex | 0.714 | f2hdlrex+cchdlrex | 0.654 |
| cdl4ldlhdl+cl rextex | 0.812 | cd14hdl+c1ldlrex | 0.714 | cd14ldltex+cchdl | 0.653 |
| cd14ldltex+f2ldlhdl | 0.812 | c1rextex+ccrex | 0.714 | cd14hdltex+g1hdl | 0.653 |
| f2ldlhdl+ccrextex | 0.812 | cd14hdl+f2hdl | 0.714 | c1ldltex+ccldlhdl | 0.653 |
| c1ldltex+f2ldlhdl | 0.812 | c1ldlhdl+f2ldltex | 0.714 | f2tex+cchdl | 0.653 |
| c1ldl+c1hdl | 0.811 | c1tex+g1tex | 0.713 | cd14rex+cchdl | 0.653 |
| c1hdl+f2hdl | 0.811 | c1ldlhdl+g1ldl | 0.713 | cchdl+ccldlrex | 0.653 |
| c1hdl+ccldlrex | 0.811 | cd14hdl+f2ldl | 0.713 | f2hdltex+ccldlhdl | 0.653 |
| cd14hdtex+g1rextex | 0.811 | c1ldlhdl+ccldl | 0.712 | g1ldltex+cchdlrex | 0.653 |
| c1hdl+f2tex | 0.811 | cd14ldl+c1tex | 0.712 | g1hdlrex+cchdltex | 0.653 |
| cdl4rextex+f2ldlhdl | 0.811 | cd14ldlrex+c1ldlhdl | 0.712 | cd14rextex+cchdl | 0.652 |
| cdl4rex+f2ldlhdl | 0.811 | c1rextex+ccldlrex | 0.712 | cd14tex+cchdltex | 0.652 |
| c1ldl+f2ldlhdl | 0.811 | cd14hdl+c1rex | 0.711 | c1ldltex+cctex | 0.652 |
| cd14hdltex+ccldltex | 0.811 | cd14hdl+g1ldl | 0.711 | g1ldl+cchdl | 0.651 |
| cd14hdl+c1hdltex | 0.81 | c1rextex+f2hdltex | 0.711 | ccrex+ccldlhdl | 0.651 |
| cd14hdl+c1hdl | 0.81 | cd14rextex+c1tex | 0.711 | f2hdl+cchdltex | 0.651 |
| c1hdl+ccldlhdl | 0.81 | cd14tex+c1tex | 0.711 | f2hdltex+cchdltex | 0.651 |
| cd14hdltex+g1ldltex | 0.809 | cd14hdl+g1rex | 0.71 | g1ldl+g1rextex | 0.651 |
| c1hdltex+cchdl | 0.808 | cd14ldltex+c1tex | 0.71 | g1hdlrex+ccldlhdl | 0.651 |
| cd14ldlhdl+g1hdltex | 0.808 | c1rex+c1tex | 0.71 | c1ldltex+cchdlrex | 0.651 |
| cd14ldl+cd14hdltex | 0.806 | c1tex+ccldlhdl | 0.71 | cctex+cchdltex | 0.65 |
| cd14ldlhdl+cd14hdltex | 0.806 | c1ldlhdl+ccldlrex | 0.709 | f2tex+ccldlhdl | 0.65 |
| cd14ldlhdl+g1ldltex | 0.806 | cd14hdl+f2rex | 0.709 | cctex+cchdlrex | 0.65 |
| c1hdl+c1ldlhdl | 0.804 | c1tex+f2rex | 0.709 | ccldl+cchdltex | 0.65 |
| cd14hdltex+g1hdltex | 0.803 | c1tex+g1ldlhdl | 0.709 | cd14rextex+g1hdltex | 0.649 |
| cd14ldlhdl+c1tex | 0.803 | cd14ldlrex+c1tex | 0.709 | g1hdl+ccldlhdl | 0.649 |
| cd14ldlhdl+g1rextex | 0.803 | c1tex+g1ldlrex | 0.709 | c1rex+ccldlhdl | 0.649 |
| c1ldl+c1ldlhdl | 0.801 | cd14ldltex+c1rextex | 0.709 | cd14ldlrex+ccldlhdl | 0.649 |
| c1hdl+f2hdltex | 0.801 | c1rextex+g1hdlrex | 0.709 | f2rex+cchdltex | 0.649 |
| cd14ldlhdl+cd14ldltex | 0.801 | f2ldl+f2rex | 0.709 | cchdl+ccldltex | 0.648 |
| cd14hdltex+g1ldlhdl | 0.8 | ccrex+cchdlrex | 0.709 | g1ldl+g1tex | 0.648 |
| c1hdl+f2ldlrex | 0.8 | g1hdl+cchdlrex | 0.708 | ccldltex+cchdlrex | 0.648 |
| c1hdl+f2ldl | 0.8 | cd14hdltex+c1tex | 0.707 | g1hdlrex+cctex | 0.648 |
| c1hdl+c1ldlrex | 0.799 | cltex+ccrex | 0.707 | cd14ldltex+cchdlrex | 0.648 |
| cd14ldlhdl+f2rextex | 0.799 | cd14hdltex+cctex | 0.707 | f2rextex+g1ldl | 0.647 |
| cd141dlhdl+ccldltex | 0.797 | c1tex+ccldltex | 0.706 | f2tex+g1ldl | 0.647 |
| cd14ldlhdl+cd14rextex | 0.797 | c1ldlhdl+f2hdl | 0.706 | g1hdl+cctex | 0.647 |
| c1hdltex+cchdlrex | 0.794 | cd14rex+c1tex | 0.706 | f2tex+f2hdlrex | 0.646 |
| cd14tex+cd14ldlhdl | 0.793 | c1ldl+c1tex | 0.706 | g1ldlhdl+ccldlhdl | 0.646 |
| cd14ldlhdl+cchdltex | 0.793 | cltex+f2ldlrex | 0.706 | f2hdltex+ccldlrex | 0.646 |
| cd14ldlhdl+g1tex | 0.792 | c1ldlrex+f2ldlrex | 0.705 | f2tex+g1tex | 0.644 |
| cd14hdlrex+c1tex | 0.791 | cd14rex+cd14hdltex | 0.704 | glhdl+g1tex | 0.644 |
| c1hdltex+f2ldl | 0.79 | c1tex+c1rextex | 0.704 | cchdl+ccldlhdl | 0.644 |
| cd14ldlhdl+cchdlrex | 0.789 | cltex+cctex | 0.704 | c1ldlrex+g1tex | 0.642 |
| c1ldlhdl+c1hdltex | 0.787 | c1rex+c1rextex | 0.703 | g1ldlhdl+cchdl | 0.642 |
| cd14hdltex+ccldlrex | 0.784 | | | | |

The three best individual markers appeared to be SerpinC1 with the ratio HDL/TEX and an AUC of 0.842 (95% Cl 0.741-0.944); SerpinC1 with the ratio HDL/REX and an AUC of 0.828 (95% Cl 0.718-0.938)) and SerpinC1 in the HDL fraction with an AUC of 0.812 (95% Cl 0.700-0.924) to discriminate between stable coronary artery disease and matched controls. Selection of the best combination of markers and sub-fractions (without the ratios of markers between the sub-fractions) using Logistics regression analysis with forward selection based upon Akaike Information Criterion (AIC) showed an AUC of 0.861 (95% Cl 0.86 - 1). The combination of markers consisted of SerpinC1-HDL + SerpinG1-TEX + SerpinC1-REX (see Figure 4) without using the ratios between the sub-fractions, because with 30 vs 30 patients one is underpowered to use all biomarkers in all combinations. This is a conservative choice that prevents overfitting of the regression model and for this gives a more realistic calculation. These results show that using the same set of extracellular protein levels in four fractions can be used in a different cohort of a different cardiovascular syndrome: stable angina, which has the same underlying disease process: atherosclerosis. Using the selected combination with an optimal cut-off value based on the AUC, it was shown that the sensitivity of these three markers was 93.3% and specificity of 76.7%. The NPV is 92 % and the Positive Predictive Value is 80 %. Figure 5 shows the highest scoring combinations of marker/sub-fraction for the Myomarker cohort but now (as for unstable angina) including the ratios of the biomarker between the plasma sub-fractions. This combination/ratios between fractions is used in the calculation of the best combination of extracellular vesicle markers after validation of the extracellular vesicle markers in the large cohorts (Example 5) that will give enough power as patient numbers are much higher.

### Example 5. Validation of extracellular vesicle proteins for diagnosis of stable angina in large cohorts

A follow up study is executed for the validation of the extracellular vesicle proteins for the diagnosis of stable angina. For this, the MYOMARKER cohort, described above, is used. This cohort contains around 1250 patients with suspected myocardial ischemia causing stable angina. It is anticipated that around 250 patients have significant myocardial ischemia on radionuclide myocardial perfusion imaging. In all patients, the 5 extracellular vesicle proteins SerpinF2, SerpinG1, SerpinC1, CD14 and Cystatin C are measured in the plasma fractions LDL, HDL, REX and TEX using sequential precipitation and an immuno-bead assay. Using back and forward selection, the AUC, NPV, PPV, sensitivity and specificity are determined in order to identify the markers (including the ratios of the biomarker between the plasma sub-fractions) and the combination of markers with the best diagnostic performance.

## Claims

1. Method for identifying a subject suffering from an ischemic heart disease, which is stable angina, said method comprising the steps of:
a) obtaining one or more of the plasma fractions selected from the group consisting of the Low-Density Lipoprotein (LDL) fraction and the High-Density Lipoprotein (HDL) fraction from a plasma sample of said subject;
b) determining one or more values, wherein each of the one or more values:
- is a value derived from a concentration of a protein marker associated with the extracellular vesicles in one of said plasma fractions, wherein the protein marker is selected from the group consisting of SerpinC1, Cystatin C, CD14, SerpinF2 and SerpinG1, wherein at least one of the one or more values is derived from a concentration of SerpinC1, or
- is a value which is a ratio of two values derived from the concentrations of a single protein marker associated with the extracellular vesicles in two different ones of said plasma fractions, wherein the protein marker is selected from the group consisting of SerpinC1, Cystatin C, CD14, SerpinF2 and SerpinG1, or
- is a value which is a combination of a value derived from a concentration of a protein marker associated with the extracellular vesicles in one of said plasma fractions, wherein the protein marker is selected from the group consisting of SerpinC1, Cystatin C, CD14, SerpinF2 and SerpinG1, and a value which is a ratio of two values derived from the concentrations of a single protein marker associated with the extracellular vesicles in two different ones of said plasma fractions, wherein the protein marker is selected from the group consisting of SerpinC1, Cystatin C, CD14, SerpinF2 and SerpinG1,
and wherein the one or more values are selected such that the area under the curve (AUC) differs from the diagonal reference line of 0.5 with a p-value of 0.05 or less, as determined by Receiver Operating Characteristic (ROC) plot analysis of the combination of said one or more values based on a suitable group of definitive subjects and group of reference subjects,
c) performing a comparison of the one or more values as determined in step b) with one or more corresponding reference values, which has been derived in the same way from the concentration of the same one or more protein marker in corresponding plasma fractions as determined in group of reference subjects not suffering from said ischemic heart disease, wherein a statistically significant difference between the one or more values determined in step b) and the one or more corresponding reference values is indicative of the subject suffering from said ischemic heart disease.

2. Method according to claim 1, wherein the one or more values in step b) are selected such that the area under the curve (AUC) is 0.8 or more and the p-value is 0.05 or less, as determined by Receiver Operating Characteristic (ROC) plot analysis of the combination of said one or more values based on a suitable group of definitive subjects and group of reference subjects.

3. Method according to any of the preceding claims, wherein the negative predictive value and/or the positive predictive value is 0.8 or more.

4. Method according to claim 1 or 2, wherein at least one of the one or more values is selected from the group consisting of values derived from the concentration of:
- SerpinC1 in the HDL plasma fraction,
and
- CD14 in the HDL plasma fraction,
and/or wherein at least one of the one or more values is a ratio of two values derived from concentrations of a single protein marker in two different ones of said plasma fractions, selected from the group consisting of:
- the concentration of SerpinC1 in the LDL plasma fraction over the concentration in the HDL plasma fraction,
- the concentration of CD14 in the LDL plasma fraction over the concentration in the HDL plasma fraction,
- the concentration of Cystatin C in the LDL plasma fraction over the concentration in the HDL plasma fraction,
- the concentration of SerpinF2 in the LDL plasma fraction over the concentration in the HDL plasma fraction.

5. Method according to claim 4, wherein at least one of the one or more values is selected from the group consisting of a value derived from the concentration of:
- SerpinC1 in the HDL plasma fraction,
and/or a value which is a ratio of
- a value derived from the concentration of SerpinF2 in the LDL plasma fraction over a value derived from the concentration in the HDL plasma fraction.

6. Method according to any of the preceding claims, wherein at least one of the one or more values is a ratio of two values derived from concentrations of a single protein marker in two different ones of said plasma fractions.

7. Method according to any of the preceding claims, wherein step b) comprises determining at least two values involving at least two different protein markers.

8. Method according to any of the preceding claims, wherein at least one protein marker is SerpinC1.

## Patentansprüche

1. Verfahren zum Identifizieren eines Individuums, das an einer ischämischen Herzerkrankung leidet, die stabile Angina pectoris ist, wobei das Verfahren die Schritte umfasst:
a) Erhalten einer oder mehrerer Plasmafraktionen, die ausgewählt sind aus der Gruppe bestehend aus der Lipoprotein-geringer-Dichte (Low-Density Lipoprotein, LDL)-Fraktion und der Lipoprotein-hoher-Dichte (High-Density Lipoprotein, HDL)-Fraktion aus einer Plasmaprobe des Individuums;
b) Bestimmen eines oder mehrerer Werte, wobei jeder der einen oder mehreren Werte:
- ein Wert ist, der abgeleitet ist aus der Konzentration eines Proteinmarkers, der mit den extrazellulären Vesikeln in einer der Plasmafraktionen assoziiert ist, wobei der Proteinmarker ausgewählt ist aus der Gruppe bestehend aus SerpinC1, Cystatin C, CD14, SerpinF2 und SerpinG1, wobei mindestens einer des einen oder der mehreren Werte abgeleitet ist aus einer SerpinC1-Konzentration, oder
- ein Wert ist, der ein Quotient zweier Werte ist, die abgeleitet sind aus den Konzentrationen eines einzelnen Proteinmarkers, der mit den extrazellulären Vesikeln in zwei verschiedenen der Plasmafraktionen assoziiert ist, wobei der Proteinmarker ausgewählt ist aus der Gruppe bestehend aus SerpinC1, Cystatin C, CD14, SerpinF2 und SerpinG1, oder
- ein Wert ist, der eine Kombination ist aus einem Wert, der abgeleitet ist aus einer Konzentration eines Proteinmarkers, der mit den extrazellulären Vesikeln in einer der Plasmafraktionen assoziiert ist, wobei der Proteinmarker ausgewählt ist aus der Gruppe bestehend aus Serpin C1, Cystatin C, CD14, SerpinF2 und SerpinG1, und einem Wert, der ein Quotient zweier Werte ist, die abgeleitet sind aus den Konzentrationen eines einzelnen Proteinmarkers, der mit den extrazellulären Vesikeln in zwei verschiedenen der Plasmafraktionen assoziiert ist, wobei der Proteinmarker ausgewählt ist aus der Gruppe bestehend aus SerpinC1, Cystatin C, CD14, SerpinF2 und SerpinG1,
und wobei der eine oder die mehreren Werte so ausgewählt ist/sind, dass sich die Fläche unter der Kurve (area under the curve, AUC) von der diagonalen Referenzlinie von 0,5 mit einem p-Wert von 0,05 oder weniger unterscheidet, wie durch Receiver Operating Characteristic (ROC)-Plotanalyse der Kombination des einen oder der mehreren Werte bestimmt, basierend auf einer geeigneten Gruppe definitiver Individuen und einer Gruppe von Referenzindividuen,
c) Durchführen eines Vergleichs des einen oder der mehreren Werte, wie in Schritt b) bestimmt, mit einem oder mehreren entsprechenden Referenzwerten, die auf die gleiche Weise von der Konzentration des gleichen einen oder der mehreren Proteinmarker in entsprechenden Plasmafraktionen, wie bestimmt, abgeleitet wurden in einer Gruppe von Referenzindividuen, die nicht an einer ischämischen Herzerkrankung leiden, wobei ein statistisch signifikanter Unterschied zwischen dem einen oder den mehreren in Schritt b) bestimmten Werten und dem einen oder den mehreren entsprechenden Referenzwerten darauf hinweist, dass das Individuum an einer ischämischen Herzerkrankung leidet.

2. Verfahren nach Anspruch 1, wobei der eine oder die mehreren Werte in Schritt b) so ausgewählt sind, dass die Fläche unter der Kurve (AUC) 0,8 oder mehr beträgt und der p-Wert 0,05 oder weniger beträgt, wie durch Receiver Operating Characteristic (ROC)-Plotanalyse der Kombination des einen oder der mehreren Werte bestimmt, basierend auf einer geeigneten Gruppe definitiver Individuen und einer Gruppe von Referenzindividuen.

3. Verfahren nach einem der vorangehenden Ansprüche, wobei der negative Vorhersagewert und/oder der positive Vorhersagewert 0,8 oder mehr sind/ist.

4. Verfahren nach Anspruch 1 oder 2, wobei mindestens einer des einen oder der mehreren Werte ausgewählt ist aus der Gruppe bestehend aus Werten, die abgeleitet sind aus der Konzentration aus:
- SerpinC1 in der HDL-Plasmafraktion,
und
- CD14 in der HDL-Plasmafraktion,
und/oder wobei mindestens einer des einen oder der mehreren Werte ein Quotient aus zwei Werten ist, die abgeleitet sind aus den Konzentrationen eines einzelnen Proteinmarkers in zwei verschiedenen der Plasmafraktionen, ausgewählt ist aus der Gruppe bestehend aus:
- der Konzentration von SerpinC1 in der LDL-Plasmafraktion geteilt durch die Konzentration in der HDL-Plasmafraktion,
- der Konzentration von CD14 in der LDL-Plasmafraktion geteilt durch die Konzentration in der HDL-Plasmafraktion,
- der Konzentration von Cystatin C in der LDL-Plasmafraktion geteilt durch die Konzentration in der HDL-Plasmafraktion,
- der Konzentration von SerpinF2 in der LDL-Plasmafraktion geteilt durch die Konzentration in der HDL-Plasmafraktion.

5. Verfahren nach Anspruch 4, wobei mindestens einer des einen oder der mehreren Werte ausgewählt ist aus der Gruppe bestehend aus einem Wert, der abgeleitet ist aus der Konzentration aus:
- SerpinC1 in der HDL-Plasmafraktion,
und/oder einem Wert, der ein Quotient ist aus
- einem Wert, der abgeleitet ist aus der Konzentration von SerpinF2 in der LDL-Plasmafraktion geteilt durch einen Wert, der abgeleitet ist aus der Konzentration in der HDL-Plasmafraktion.

6. Verfahren nach einem der vorangehenden Ansprüche, wobei mindestens einer des einen oder der mehreren Werte ein Quotient ist aus zwei Werten, die abgeleitet sind aus den Konzentrationen eines einzelnen Proteinmarkers in zwei verschiedenen Plasmafraktionen.

7. Verfahren nach einem der vorangehenden Ansprüche, wobei Schritt b) das Bestimmen mindestens zweier Werte umfasst, die mindestens zwei verschiedene Proteinmarker involvieren.

8. Verfahren nach einem der vorangehenden Ansprüche, wobei mindestens ein Proteinmarker SerpinC1 ist.

## Revendications

1. Procédé d'identification d'un sujet souffrant d'une cardiopathie ischémique, qui est une angine stable, ledit procédé comprenant les étapes consistant à :
a) obtenir une ou plusieurs des fractions de plasma sélectionnées dans le groupe consistant en la fraction de lipoprotéines basse densité (LDL) et la fraction de lipoprotéines haute densité (HDL) à partir d'un échantillon de plasma dudit sujet ;
b) déterminer une ou plusieurs valeurs, dans lequel chacune des une ou plusieurs valeurs :
- est une valeur dérivée d'une concentration d'un marqueur protéique associé aux vésicules extracellulaires dans l'une desdites fractions de plasma, dans lequel le marqueur protéique est sélectionné dans le groupe consistant en la serpine C1, la cystatine C, CD14, la serpine F2 et la serpine G1, dans lequel au moins l'une des une ou plusieurs valeurs est dérivée d'une concentration de serpine C1, ou
- est une valeur qui est un rapport de deux valeurs dérivées des concentrations d'un seul marqueur protéique associé aux vésicules extracellulaires dans deux différentes desdites fractions de plasma, dans lequel le marqueur protéique est sélectionné dans le groupe consistant en la serpine C1, la cystatine C, CD14, la serpine F2 et la serpine G1, ou
- est une valeur qui est une combinaison d'une valeur dérivée d'une concentration d'un marqueur protéique associé aux vésicules extracellulaires dans l'une desdites fractions de plasma, dans lequel le marqueur protéique est sélectionné dans le groupe consistant en la serpine C1, la cystatine C, CD14, la serpine F2 et la serpine G1, et d'une valeur qui est un rapport de deux valeurs dérivées des concentrations d'un seul marqueur protéique associé aux vésicules extracellulaires dans deux différentes desdites fractions de plasma, dans lequel le marqueur protéique est sélectionné dans le groupe consistant en la serpine C1, la cystatine C, CD14, la serpine F2 et la serpine G1,
et dans lequel les une ou plusieurs valeurs sont sélectionnées de façon que la surface sous la courbe (SSC) diffère de la ligne diagonale de référence de 0,5 avec une valeur p de 0,05 ou moins, tel que déterminé par une analyse graphique de caractéristique de fonctionnement du récepteur (ROC) de la combinaison desdites une ou plusieurs valeurs sur la base d'un groupe approprié de sujets définitifs et d'un groupe de sujets de référence,
c) mettre en œuvre une comparaison des une ou plusieurs valeurs telles que déterminées dans l'étape b) avec une ou plusieurs valeurs de référence correspondantes, qui ont été dérivées de la même manière à partir de la concentration du même ou des mêmes un ou plusieurs marqueurs protéiques dans des fractions de plasma correspondantes telles que déterminées dans un groupe de sujets de référence ne souffrant pas de ladite cardiopathie ischémique, dans lequel une différence statistiquement significative entre les une ou plusieurs valeurs déterminées dans l'étape b) et les une ou plusieurs valeurs de référence correspondantes indique que le sujet souffre de ladite cardiopathie ischémique.

2. Procédé selon la revendication 1, dans lequel les une ou plusieurs valeurs dans l'étape b) sont sélectionnées de façon que la surface sous la courbe (SSC) soit de 0,8 ou plus et que la valeur p soit de 0,05 ou moins, tel que déterminé par une analyse graphique de caractéristique de fonctionnement du récepteur (ROC) de la combinaison desdites une ou plusieurs valeurs sur la base d'un groupe approprié de sujets définitifs et d'un groupe de sujets de référence.

3. Procédé selon l'une quelconque des revendications précédentes, dans lequel la valeur prédictive négative et/ou la valeur prédictive positive est de 0,8 ou plus.

4. Procédé selon la revendication 1 ou 2, dans lequel au moins l'une des une ou plusieurs valeurs est sélectionnée dans le groupe consistant en les valeurs dérivées de la concentration de :
- la serpine C1 dans la fraction de plasma HDL,
et
- CD14 dans la fraction de plasma HDL,
et/ou dans lequel au moins l'une des une ou plusieurs valeurs est un rapport de deux valeurs dérivées de concentrations d'un seul marqueur protéique dans deux différentes desdites fractions de plasma, sélectionnées dans le groupe consistant en :
- la concentration en serpine C1 dans la fraction de plasma LDL sur la concentration dans la fraction de plasma HDL,
- la concentration en CD14 dans la fraction de plasma LDL sur la concentration dans la fraction de plasma HDL,
- la concentration en cystatine C dans la fraction de plasma LDL sur la concentration dans la fraction de plasma HDL,
- la concentration en serpine F2 dans la fraction de plasma LDL sur la concentration dans la fraction de plasma HDL.

5. Procédé selon la revendication 4, dans lequel au moins l'une des une ou plusieurs valeurs est sélectionnée dans le groupe consistant en une valeur dérivée de la concentration :
- de la serpine C1 dans la fraction de plasma HDL,
et/ou une valeur qui est un rapport
- d'une valeur dérivée de la concentration en serpine F2 dans la fraction de plasma LDL sur une valeur dérivée de la concentration dans la fraction de plasma HDL.

6. Procédé selon l'une quelconque des revendications précédentes, dans lequel au moins l'une des une ou plusieurs valeurs est un rapport de deux valeurs dérivées de concentrations d'un seul marqueur protéique dans deux différentes desdites fractions de plasma.

7. Procédé selon l'une quelconque des revendications précédentes, dans lequel l'étape b) comprend la détermination d'au moins deux valeurs impliquant au moins deux marqueurs protéiques différents.

8. Procédé selon l'une quelconque des revendications précédentes, dans lequel au moins un marqueur protéique est la serpine C1.
